# EUROPEAN PATENT SPECIFICATION

(11) **EP 3 701 038 B1**
(45) Date of publication and mention of the grant of the patent: **09.10.2024**
(21) Application number: 18788777.3
(22) Date of filing: 24.10.2018
(51) Int. Cl.: C12Q 1/04, B33Y 10/00, C12N 1/20

(54) **METHODS FOR PERFORMING CELL CULTURE**
VERFAHREN ZUR DURCHFÜHRUNG EINER ZELLKULTUR
PROCÉDÉS POUR RÉALISER LA CULTURE DE CELLULES

(30) Priority: 26.10.2017 EP 17198564
(43) Date of publication of application: 02.09.2020
(73) Proprietor: Merck Patent GmbH, 64293 Darmstadt (DE)
(72) Inventor: STOCKUM, Werner, 64354 Reinheim (DE); LINDHARDT, Charlotte, 64297 Darmstadt (DE)
(74) Representative: Merck Patent Association
(86) International application number: PCT/EP2018/079078
(87) International publication number: WO 2019/081540

(56) References cited:
- FR-A1- 2 559 499
- MASAYUKI UCHIDA ET AL: "Safety of high doses ofET-3 culture in healthy adult subjects", REGULATORY TOXICOLOGY AND PHARMACOLOGY, vol. 60, no. 2, 21 December 2010 (2010-12-21), pages 262 - 267, XP028376776, ISSN: 0273-2300, [retrieved on 20101221], DOI: 10.1016/J.YRTPH.2010.12.005
- KEVIN ALESSANDRI ET AL: "A 3D printed microfluidic device for production of functionalized hydrogel microcapsules for culture and differentiation of human Neuronal Stem Cells (hNSC)", LAB ON A CHIP, vol. 16, no. 9, 1 January 2016 (2016-01-01), pages 1593 - 1604, XP055393107, ISSN: 1473-0197, DOI: 10.1039/C6LC00133E
- DAVID M. KINGSLEY ET AL: "Microcapsules and 3D customizable shelled microenvironments from laser direct-written microbeads : Microcapsules and 3D Customizable Shelled Microenvironments", BIOTECHNOLOGY AND BIOENGINEERING, vol. 113, no. 10, 1 October 2016 (2016-10-01), US, pages 2264 - 2274, XP055524656, ISSN: 0006-3592, DOI: 10.1002/bit.25987
- CHRISTOPH BADER ET AL: "Grown, Printed, and Biologically Augmented: An Additively Manufactured Microfluidic Wearable, Functionally Templated for Synthetic Microbes", 3D PRINTING AND ADDITIVE MANUFACTURING, vol. 3, no. 2, 1 June 2016 (2016-06-01), pages 79 - 89, XP055524746, ISSN: 2329-7662, DOI: 10.1089/3dp.2016.0027
- ZHIKAI TAN ET AL: "Control of cell growth on 3D-printed cell culture platforms for tissue engineering", JOURNAL OF BIOMEDICAL MATERIALS RESEARCH. PART A, vol. 105, no. 12, 15 September 2017 (2017-09-15), HOBOKEN, NY, US, pages 3281 - 3292, XP055525134, ISSN: 1549-3296, DOI: 10.1002/jbm.a.36188

## Description

The present invention relates to methods for performing cell culture by using a solid form of a cell culture mediumprepared by a 3D printing process.

Cell culture media are available for the growth and culture of cells like mammalian cells, plant cells, bacteria, yeast and moulds for a long time. Cell culture media may comprise of a complex mixture of components, sometimes more than one hundred different components, depending on the type of organism whose growth and/or targeted physiological status shall be supported. The cell culture media required for the propagation of mammalian, insect or plant cells are typically much more complex than the media to support the growth of bacteria, yeast or fungi.

The first cell culture media that were developed consisted of undefined components, such as plasma, serum, embryo extracts, or other non-defined biological extracts or peptones. A major advance was thus made with the development of chemically defined media. Chemically defined media often comprise but are not exclusively limited to amino acids, vitamins, metal salts, antioxidants, chelators, growth factors, buffers, hormones, and many more substances known to those expert in the art.

Some cell culture media are offered as sterile aqueous liquids. The disadvantage of liquid cell culture media is their reduced shelf life and difficulties for shipping and storage. As a consequence, many cell culture media are presently offered as finely milled dry powder mixtures. They are manufactured for the purpose of dissolving in water and/or aqueous solutions and in the dissolved state are designed, often with other supplements, for supplying cells with a substantial nutrient base for growth and/or production of biopharmaceuticals from same said cells.

Ideally, all components of the cell culture medium are mixed either in solid state to be dissolved prior to use or in a ready-to-use liquid state. But this is often not possible.

A limiting factor for the preparation and the use of cell culture media is the poor stability of some components, which tend to e.g. degrade and/or or oxidize during storage or when being autoclaved. Antibiotics and vitamins are often heat sensitive and need to be added after autoclaving. Some components are hygroscopic which often results in clumping of a dry powder medium leading to stability and/or other quality problems restricting their industrial application(s). The water attracted by the hygroscopic compounds might lead to increased microbial growth as well as degradation of neighbouring components. In addition, certain components might show cross-reactivity with other components leading to unwanted side-reactions and change of the media composition.

Another limitation of producing pre-mixed media is a lack of flexibility. It might be favourable to have a basic media composition which is then supplemented with certain components as needed.

In all of these cases there is typically a cell culture base medium in either liquid or dry state which is prior to use or at certain times during cell culture supplemented with cell culture media supplements comprising one or more components which are also needed for the cell culture but are - e.g. due to stability or strategic reasons - not part of the base medium.

Adding those media supplements is often complicated and time consuming. While the composition of the base medium is fixed and the only step that might have to be performed prior to use is dissolving of the dry medium in a defined amount of solvent, more effort is needed for the addition of the supplement. It might also have to be dissolved in a certain amount of liquid and the technician needs to take care that the time of application as well as the amount and composition that is added is suitable and that the addition to the base medium or the cell culture does not cause unwanted side effects.

Also the preparation of such media supplement is often inconvenient. Media supplements are often mixes of several compounds with different solubility and are normally manufactured by dispensing of one or more separate solutions into glass vials followed by lyophilization.

Before use, the lyophilized material is reconstituted (or brought into suspension) and subsequently the solution is added to the already sterilized medium or the cell culture. The supplement solution may be filter sterilized prior to addition depending on criticality. Depending on solubility of individual supplements, the volume of liquid required for freeze drying may be large. Certain compounds may not be readily soluble in solvents acceptable for freeze drying (solvent required may be ethanol or DMSO). The same compound may be used in a number of different supplement mixes, but the base solution for manufacturing must be produced fresh for each manufacturing run.

Currently, supplement vials are typically produced only for 500 ml media volumes, which is highly inconvenient/unsuitable for larger scale (5-20 l) production of media using Media cooker automats.

It would thus be favorable to find a way to make the use and production of media supplements as easy as possible and on the other hand provide all necessary flexibility to the user.

FR 2 559 499 discloses culture media in form of a pressed tablet.

Masayuki Uchida et al., 2010, Regulatory Toxicology and Pharmacology Vol. 60, No. 2, pages 262-267, describe a culture medium tablet for human consumption.

Kevin Alessandri et al., 2016, Lab on a Chip, Vol. 16, No. 9, pages 1593-1604, disclose a 3D printed microfluidic device for the production of hydrogel microcapsules for culture of human Neuronal Stem Cells.

David M. Kingsley et al., 2016, Biotechnology and Bioengineering, Vol. 113, No. 10, pages 2264-2274, disclose laser-fabricated and patterned capsules or beads as microenvironments for growing cells.

Christoph Bader et al., 2016, 3D Printing and Additive Manufacturing Vol. 3, No. 2, pages 79-89 describe the production of 3D printed microfluidic devices for culturing cells.

Zhikai Tan et al., 2017, Journal of Biomedical Materials Research, Part A, Vol. 105, No. 12, pages 3281-3291 disclose 3D printed cell culture platforms for tissue engineering.

It has been found that it is possible to use 3D printing technologies to generate solid media forms. The composition of the solid cell culture media forms can be defined very precisely when doing the 3D printing. The 3D printed solid forms, like tablets, can then be easily applied to the cell culture, either by pre-mixing it with a solvent or by adding to a liquid cell culture in solid form. It is even possible to influence the stability of certain ingredients as well as the release of certain ingredients to e.g. generate sustained release media.

The present invention is thus directed to a process for culturing cells according to claim 1 including
a) generating a solid form of a cell culture medium by using 3D printing technologies
b) mixing said solid form with a liquid and the cells to be cultured, thereby dissolving the solid form
c) performing cell culture by incubating the mixture of step b)

The solid cell culture media form comprises at least one cell culture media ingredient.

In a preferred embodiment, the solid form is a tablet.

In another embodiment, the solid form comprises at least two layers with different composition.

In one embodiment, the solid form comprises a core and a shell with differing composition.

In another embodiment, a part of the solid form is dissolved slower compared to another part of the solid form. The part that is dissolved slower is typically the core which due to its composition provides for sustained release of its ingredients.

In a preferred embodiment, the solid form comprises 4-methylumbilliferyl-phosphate disodium, Acriflavine, Amphotericin B, Ammonium-Iron(III)-citrate, Brilliant green, Calcium carbonate, Cefiximide, Cefoperazone, Cefotetan, Cefsulodin, Ceftazimide, Cephalotin, Cetrimide, Colistin sulfate, Cyclohexidine, D-cycloserine, Fosfomycin, Fucidin, Irgasan, L-α-Phosphatidylinositol, Lithium mupirocin, Nalidixic acid, Novobiocin, Ox bile, Oxytetracycline, Polymyxin B sulfate, Potassium tellurite, Potassium tetrathionate, Rifampicin, Trimethoprim lactate salt or Vancomycin or combinations thereof.

In another preferred embodiment, the solid form comprises one or more amino acids and/or vitamins. Preferred amino acids are cysteine, tyrosine, isoleucine, leucine, valine, tryptophan, histidine and methionine. Preferred vitamins are riboflavin, thiamine, folic acid and vitamin B12.

In one embodiment, step b) is performed by first mixing said solid form with a liquid and then adding the cells. In this case the liquid might e.g. be water or an aqueous buffer or any type of liquid cell culture medium. It is also possible, at this stage, to add further components like e.g. a gelling agent before addition of the cells. The cells are typically present in a liquid cell culture medium.

In one embodiment, step b) is performed by mixing said solid form with a liquid that already comprises the cells. In this case, the cells are typically already present in a liquid cell culture medium. The addition of the solid form might take place directly after inoculation of the liquid cell culture medium with the cells or any time during culturing the cells in the liquid cell culture medium.

In one embodiment, the cell culture is a fed batch cell culture.

In another embodiment, the cell culture is a cell culture for detection and/or enumeration of certain cells. Such type of cell culture is typically used in microbiological applications.

In a preferred embodiment, the 3D printing technology used in step a) is a contactless 3D laser printing process.

In a very preferred embodiment, the printing process used in step a) comprises the steps
(a) positioning a composite layer (3) comprising a laser energy absorbing layer (1) and a layer that contains at least one cell culture media ingredient (2) between a plate (4) that is permeable for a laser beam that can be activated by a source of laser energy (5) and a mounting plate (6) whereat the layer of the composite containing at least one cell culture media ingredient (2) is positioned opposite to the source of laser energy (5) and is facing to the mounting plate (6);
(b) lowering the mounting plate (6) to shape an interspace (8) between the composite layer (3) comprising a cell culture media ingredient containing layer (2) and the mounting plate (6);
(c) transferring by action of laser beam from the source of laser energy (5) the cell culture media ingredient containing layer (2) of the composite (3) onto the mounting plate (6);
(d) repeating steps (a), (b) and (c) as often as needed to build up the solid form of a cell culture medium;
(e) removing the solid form of a cell culture medium from the mounting plate.

In another very preferred embodiment, the printing process used in step a) comprises the steps
(a) positioning a composite layer (3) comprising a laser energy absorbing layer (1) and a layer that contains at least one cell culture media ingredient (2) between between a plate (4) that is permeable for a laser beam that can be activated by a source of laser energy (5) and a mounting plate (6) comprising at least one area (7) that is movable in vertical direction (z axis) relative to the mounting plate whereat the layer of the composite containing at least one cell culture media ingredient (2) is positioned opposite to the source of laser energy (5) and is facing to the mounting plate (6);
(b) lowering the movable area (7) relative to the mounting plate (6) to shape an interspace (8') between the composite layer (3) comprising a cell culture media ingredient containing layer (2) and the movable area (7) of the mounting plate (6);
(c) transferring by action of laser beam from the source of laser energy (5) the cell culture media ingredient containing layer (2) of the composite (3) into the interspace (8') shaped by the movable area (7) that was lowered vertically relative to the mounting plate (6);
(d) repeating steps (a), (b) and (c) as often as needed to build up the solid cell culture media form;
(e) removing the solid cell culture media form from the mounting plate.

A cell culture is any setup in which cells are cultured.

A cell culture can be performed in any container suitable for the culture of cells, such as a petri dish, contact plate, bottle, tube, well, vessel, bag, flask and/or tank. Typically, the container is sterilized prior to use. A cell culture is typically performed by incubation of the cells in an aqueous cell culture medium under suitable conditions for growth and/or maintenance of the cells such as suitable temperature, osmolality, aeration, agitation, etc. which limit contamination with foreign microorganisms from the environment. A person skilled in the art is aware of suitable incubation conditions for culturing of cells.

A cell culture medium (synonymously used: culture medium) is any mixture of components which maintains and/or supports the *in vitro* growth of cells and/or supports or maintains a particular physiological state. It is also suitable for pre-enrichment cultures as well as for use as a maintenance medium.

It might comprise undefined components, such as plasma, serum, embryo extracts, or other non-defined biological extracts or peptones. It might also be a chemically defined medium. The cell culture medium can comprise all components necessary to maintain and/or support the *in vitro* growth of cells or be used for the addition of selected components in combination with or not in combination with further components that are added separately (media supplement). Preferably, the cell culture medium is a media supplement. The components of a cell culture medium are also called cell culture media ingredients.

The cell culture media and processes used in the present invention are designed to be suitable to grow or maintain/support the growth of prokaryotic cells like bacterial cells as well as eukaryotic cells like yeast, fungi, algae, plant, insect and/or mammalian cells and, optionally, archaea. The prokaryotes and eukaryotes as well as the optional archaea are in the following also called microorganisms or cells.

Microorganisms whose growth shall be maintained or supported by the methods and media of the present invention are typically found in:
- environmental samples during environmental monitoring of pharmaceutical relevance
- samples obtained from raw materials, intermediates and/or finished goods of pharmaceutical relevance
- clinical samples during hospital examinations
- veterinary samples
- water samples for examination of drinking and/or waste water and/or swimming pool water
- food samples for the examination of microbial contamination
- cosmetic samples
- cell culture for biopharmaceutical production

For example, the cell culture medium used in the present invention may support the growth of eukaryotes and prokaryotes like Gram-positive microorganisms and Gram-negative microorganisms, such as human skin contaminants, water contaminants, yeast and mould. Examples of cells of which growth is maintained/ supported/ detected by the media and methods according to the present invention are:
*Escherichia coli*/STEC/EHEC, *Salmonella* spp, *Listeria* spp, *Campylobacter* spp, *Bacillus* spp, *Cronobacter sakazakii, Vibrio* spp, *Yersinia* spp, *Legionella* spp, *Mycobacterium* spp, *Clostridium* spp, *Staphylococcus aureus, Candida* spp, *Aspergillus* spp.

Chemically defined cell culture media are cell culture media comprising of chemically well characterized 'defined' raw materials. This means that the chemical composition of all the chemicals used in the media is known. The chemically defined media do not comprise of chemically ill-defined substances like chemically ill-defined yeast, animal or plant tissues; they do not comprise peptones, feeder cells, serum, ill-defined extracts or digests or other components which may contribute chemically poorly defined proteins and/or peptides and/or hydrolysates to the media. In some cases the chemically defined medium may comprise proteins or peptides which are chemically defined - one example is insulin (see others below).

A powdered cell culture medium or a dry powder medium or a dehydrated culture medium is a cell culture medium typically resulting from a milling process or a lyophilisation process. That means the powdered cell culture medium is typically a finely granular, particulate medium - not a liquid medium. The term "dry powder" may be used interchangeably with the term "powder;" however, "dry powder" as used herein simply refers to the gross appearance of the granulated material and is not intended to mean that the material is completely free of complexed or agglomerated solvent unless otherwise indicated. A powdered cell culture medium can also be a granulated cell culture medium, e.g. dry granulated by roller compaction or wet granulated by fluidized bed spray granulation. Such a medium can also be prepared by spray drying.

A solid form of a cell culture medium, also called solid form or solid cell culture media form, is any defined 3-dimensional body resulting from a 3D printing process comprising at least one cell culture media ingredient. It is a dry solid, however, "dry solid" as used herein simply refers to the gross appearance of the material and is not intended to mean that the material is completely free of complexed or agglomerated solvent unless otherwise indicated. The solid form can be porous or non-porous. It can have any shape adapted to the application requirements, e.g. round, oval, rod like, torpedo shaped etc. Examples of solid cell culture media forms are tablets or pills.

Solvents, also called liquids, used to prepare a liquid cell culture medium are typically water (most particularly distilled and/or deionized water or purified water or water for injection) or an aqueous buffer. The solvent may also comprise saline, soluble acid or base ions providing a suitable pH range (typically in the range between pH 1 and pH 10), stabilizers, surfactants, preservatives, and alcohols or other polar organic solvents as well as gelling agents for the production of semi-solid media.

The pH of the dissolved medium prior to addition of cells is typically between pH 2 and 12, more preferable between pH 4 and 10, even more preferably between pH 6 and 8 and most preferable between pH 6.5 to 7.5 and ideally between pH 7.0 to 7.5.

A cell culture medium which comprises all components necessary to maintain and/or support the *in vitro* growth of cells typically comprises at least one or more saccharide components, one or more amino acids, one or more vitamins or vitamin precursors, one or more salts, one or more buffer components, one or more co-factors and one or more nucleic acid components (nitrogenous bases) or their derivatives. It may also comprise chemically defined biochemicals such as recombinant proteins, e.g. rlnsulin, rBSA, rTransferrin, rCytokines etc..

The media may also comprise sodium pyruvate, highly purified and hence chemically well-defined extracts, fatty acids and/or fatty acid derivatives and/or pluronic product components (block copolymers based on ethylene oxide and propylene oxide) in particular Poloxamer 188 sometimes called Pluronic F 68 or Kolliphor P 188 or Lutrol F 68 and/or surface active components such as chemically prepared non-ionic surfactants. One example of a suitable non-ionic surfactants are difunctional block copolymer surfactants terminating in primary hydroxyl groups also called poloxamers, e.g. available under the trade name pluronic ^{®} from BASF, Germany. Such pluronic product components are in the following just called pluronic. Chelators, hormones and/or growth factors may also be added.

Other components it may comprise of are the pure compounds, salts, conjugates, and/or derivatives of lactic acid, thioglycollic acid, thiosulphates, tetrathionate, diaminobutane, myo-inositol, phosphatidylcholine (lecithin), sphingomyelin, iron containing compounds (including compounds with iron sulphur clusters), uric acid, carbamoyl phosphate, succinic acid, thioredoxin(s), orotic acid, phosphatidic acid, polyamines (such as putrescine, spermidine, spermine and/or cadaverine), triglycerides, steroids (including but not limited to cholesterol), metallothionine, oxygen, glycerol, urea, alpha-ketoglutarate, ammonia, glycerophosphates, starch, glycogen, glyoxylate, isoprenoids, methanol, ethanol, propanol, butanol, acetone, lipids (including but not limited to those in micelles), tributyrin, butyrin, cholic acid, desoxycholic acid, polyphosphate, acetate, tartrate, malate and/or oxalate.

Saccharide components are all mono- or di-saccharides, like glucose, galactose, ribose or fructose (examples of monosaccharides) or sucrose, lactose or maltose (examples of disaccharides) or derivatives thereof like sugar alcohols. Saccharide components may also be oligo- or polysaccharides.

Examples of amino acids are particularly the proteinogenic amino acids, especially the essential amino acids, leucine, isoleucine, lysine, methionine, phenylalanine, threonine, tryptophan and valine, as well as the non-proteinogenic amino acids such as D-amino acids.

Tyrosine means L- or D- tyrosine, preferably L-tyrosine.

Cysteine means L- or D-cysteine, preferably L-cysteine.

Amino acid precursors and analogues are also included.

Examples of vitamins are Vitamin A (Retinol, retinal, various retinoids, and four carotenoids), Vitamin B₁ (Thiamine), Vitamin B₂ (Riboflavin), Vitamin B₃ (Niacin, niacinamide), Vitamin B₅ (Pantothenic acid), Vitamin B₆ (Pyridoxine, pyridoxamine, pyridoxal), Vitamin B₇ (Biotin), Vitamin B₉ (Folic acid, folinic acid), Vitamin B₁₂ (Cyanocobalamin, hydroxycobalamin, methylcobalamin), Vitamin C (Ascorbic acid) (including phosphates of ascorbic acid), Vitamin D (Ergocalciferol, cholecalciferol), Vitamin E (Tocopherols, tocotrienols) and Vitamin K (phylloquinone, menaquinones). Vitamin precursors and analogues are also included.

Examples of salts are components comprising inorganic ions such as bicarbonate, calcium, chloride, magnesium, phosphate, potassium and sodium or trace elements such as Co, Cu, F, Fe, Mn, Mo, Ni, Se, Si, Ni, Bi, V and Zn. Examples are copper(II) sulphate pentahydrate (CuSO₄·5 H₂O), sodium chloride (NaCl), calcium chloride (CaCl₂·2 H₂O), potassium chloride (KCI), iron(II)sulphate, sodium phosphate monobasic anhydrous (NaH₂PO₄), magnesium sulphate anhydrous (MgSO₄), sodium phosphate dibasic anhydrous (Na₂HPO₄), magnesium chloride hexahydrate (MgCl₂·6 H₂O), zinc sulphate heptahydrate (ZnSO₄·7 H₂O).

Examples of buffers are carbonate, citrate, phosphate, HEPES, PIPES, ACES, BES, TES, MOPS and TRIS.

Examples of cofactors are compounds, salts, complexes and/or derivatives of thiamine, biotin, vitamin C, calciferol, choline, NAD/NADP (reduced and/or oxidized), cobalamin, vitamin B12, flavin mononucleotide and derivatives, flavin adenine dinucleotide and derivatives, glutathione (reduced and/or oxidized and/or as dimer), haeme, haemin, haemoglobin, ferritin, nucleotide phophates and/or derivatives (e.g. adenosine phosphates), coenzyme F420, s-adenosyl methionine, coenzyme B, coenzyme M, coenzyme Q, acetyl Co-A, molybdopterin, pyrroloquinoline quinone, tetrahydrobiopterin.

Nucleic acid components are the nucleobases, like cytosine, guanine, adenine, thymine, uracil, xanthine and/or hypoxanthine, the nucleosides like cytidine, uridine, adenosine, xanthosine, inosine, guanosine and thymidine, and the nucleotides such as adenosine monophosphate or adenosine diphosphate or adenosine triphosphate, including but not limited to the deoxy- and/or phosphate derivatives and/or dimers, trimers and/or polymers thereof, like RNA and/or DNA.

Components may be added which improve the physico-chemical properties of the media, like but not limited to, increasing clarity and/or solubility of the media and/or one or more of its components, without significantly negatively affecting the cell growth properties at the concentrations used. Such components include but are not limited to chelating agents (e.g. EDTA), antioxidants, detergents, surfactants, emulsifiers (like polysorbate 80), neutralising agents, (like polysorbate 80), micelle forming agents, micelle inhibiting agents and/or polypropylene glycol, polyethylene alcohol and/or carboxymethylcellulose.

The medium typically contains carbohydrates such as sugars and/or sugar mixtures and/or sugar dimers and/or sugar polymers and/or their derivatives. Typically, glucose and/or lactose and/or galactose can be the main carbohydrate sugar components. Glucose is usually included in the medium at a concentration of 0,001 mM to 250 mM in the aqueous medium solution, more preferably 1mM to 100 mM, even more preferably 5 mM to 50 mM.

Typically, the medium comprises each amino acid in a range from 10 mg to 3g per liter, preferably in a range from 40 mg to 1 g per liter of the liquid medium.

The medium typically comprises vitamins. A typical amount of a vitamin in the medium is in the range of 5 µg to 10 mg per liter, preferably in the range of 50 µg to 6 mg per liter of the liquid medium.

Typically, the medium comprises salts. The amount of one type of salt is typically in the range of 2 µg to 5 mg per liter, preferably in the range of 10 µg to 1.5 mg per liter of the liquid medium. Specific salts may also be present in much higher amounts; the concentration of NaCl can for example be up to 30 g per liter of the liquid medium.

The typical amount of a nucleic acid comprised in the medium is in the range of 0.5 to 10 mg per liter, preferably in the range of 1 to 5 mg per liter of the liquid medium.

The medium typically contains all the proteogenic amino acids (and/or their derivatives and/or their conjugates and/or dimers (pure and/or mixed) thereof).

Other defined components may also be added to aid detection or identification of microorganisms like indicators, e.g. a pH indicator.

The culture medium can further comprise at least one chromogenic or fluorogenic substrate. Fluorogenic substrates are complex molecules which, on contact with enzymes synthesized by microorganisms, are cleaved and become fluorescent. The fluorescence emitted is detectable visually and/or with an analytical instrument like a spectrophotometer by illuminating the growth medium using radiation in the UV or visible spectrum. Examples of fluorogenic substrates are fluorescein derivatives (CFA, CFDA), methylumbelliferone derivatives or the Aldols^{™} (developed by the company Biosynth).

Chromogenic substrates are substrates that change their color when they are modified e.g. by a specific enzyme of a microorganism. Examples of chromogenic substrates are ONPG (Ortho-nitrophenyl-β-D-galactopyranoside) or X-Gal (5-bromo-4-chloro-3-indolyl-beta-D-galactopyranoside. In this case the medium color after the growth of certain microorganisms of interest will change color and be indicative of such microorganisms.

A typically suitable liquid cell culture medium has a typical composition of 2 to 50 g/L, more preferably 5 to 30 g/L. A medium with a gelling agent has typically an additional weight due to the gelling agent of between 1 and 50 g/L, more preferably between 2 and 30 g/L.

The osmolality of the medium is typically between 50 mOsm and 1000 mOsm, more preferably between 150 mOsm and 500 mOsm.

Media supplements are cell culture media that are added to a cell culture base medium prior to starting a cell culture or to a cell culture one or more times during cell culture. Media supplements that are added during cell culture are typically added in liquid format. This is often due to the fact that powders don't dissolve easily without stirring or agitation. But during cell culture stirring or agitation might not be possible as it causes distraction of the cells. It has been found the 3D printed solid cell culture media forms generated during the process of the present invention are suitable for being added in solid form even to a running cell culture. The dissolution properties of a 3D printed tablet can be designed e.g. by using certain additives that lead to fast or slow dissolution and/or by 3D printing solid forms with a certain structure that also influences the dissolution behavior of the solid form.

Media supplements are often used in mammalian cell culture like CHO cell culture for production of proteins that form the basis of biological drugs. Advantages of using media supplements for this application include customizing the growth conditions of the cells, improving cell viability and growth, and keeping cells healthier for a longer time.

Examples of media supplements that are used for mammalian cell culture are 2-mercaptoethanol, supplements comprising lipids, supplements comprising one or more amino acids, supplements comprising one or more vitamins, bovine serum albumin, pluronic F68, cholesterol, sodium pyruvate, glutamine, transferrin and insulin. Especially preferred are supplements comprising one or more amino acids like, preferably, cysteine, tyrosine, leucine, isoleucine, valine, tryptophan, histidine and/or methionine and/or one or more vitamins like riboflavin, thiamine, folic acid and/or vitamin B12.

Supplements to be used in e.g. microbiological applications are for example supplements which support the growth of all or selected cells, antibiotic supplements or chromogenic supplements. Examples of

supplements are 4-methylumbilliferyl-phosphate disodium, Acriflavine, Amphotericin B, Ammonium-Iron(III)-citrate, Brilliant green, Calcium carbonate, Cefiximide, Cefoperazone, Cefotetan, Cefsulodin, Ceftazimide, Cephalotin, Cetrimide, Colistin sulfate, Cyclohexidine, D-cycloserine, Fosfomycin, Fucidin, Irgasan, L-α-Phosphatidylinositol, Lithium mupirocin, Nalidixic acid, Novobiocin, Ox bile, Oxytetracycline, Polymyxin B sulfate, Potassium tellurite, Potassium tetrathionate, Rifampicin, Trimethoprim lactate salt or Vancomycin or combinations thereof, most preferred are Acriflavine, Amphotericin B, Cefoperazone, Cefsulodin, Ceftazimide, Cetrimide, Cyclohexidine, Nalidixic acid, Novobiocin, Polymyxin B sulfate, Potassium tellurite, Trimethoprim lactate salt or Vancomycin or combinations thereof.

The supplements can be present in the solid form as single components or as mixtures of one or more of the supplement components mentioned above, as mixtures with one or more other cell culture media ingredients listed above or with any other components deemed to be suitable for cell culture.

3D Printing, also known as Additive Manufacturing, refers to processes used to create a three-dimensional solid object whereby layers of material are formed under computer control based on a digital file to create said object. The creation of a 3D printed object is achieved using so called additive processes. In an additive process an object is created by laying down successive layers of material until the object is created. Each of these layers can be seen as a thinly sliced horizontal cross-section of the eventual object. 3D printing is the opposite of subtractive manufacturing which is cutting out / hollowing out a piece of metal or plastic with for instance a milling machine.

Several techniques for 3D printing are known to a person skilled in the art. Depending on the component or mixture of components to be printed, certain 3D printing techniques might be more suitable than others. A person skilled in the art of 3D printing can choose a suitable 3D printing technique for a given composition.

Examples of suitable 3D printing techniques are Fused Deposition Modeling, Polyjet 3D printing, Binder jetting, Vat polymerization or Selective Laser Sintering.

In fused deposition modeling, also called fused filament (FF) fabrication and paste extrusion, filaments of the composition to be 3D printed are molten and dispensed through a nozzle.

The composition, typically a polymer strand, is heated and extruded through a small tip (typically 50-100 □m) and then solidified on a build plate. FF technology has the significant advantages of cost (typical systems cost between £800-2000), the ability to fabricate hollow objects and the utility to print a range of polymers. The printer feedstock is an extruded polymer filament, typically 1.75 - 3 mm in diameter. One of the prime benefits of FF 3DP is that it is possible in principle to incorporate compounds into the polymer filament so that the printed dosage form is loaded with said compounds.

Further details about the technology are known to a person skilled in the art and can e.g. be found in Goyanes et al. Int J Pharm. 2014 Dec 10;476(1-2):88-92.

In Polyjet 3D printing, also called multi jet 3D printing, droplets of build and support materials are selectively jetted and cured by UV radiation. The use of multiple nozzles simultaneously can improve the printing speed of this process over other techniques, such as powder bed 3D printing and fused deposition modelling. The combination of fast printing speeds with rapid polymerization upon short exposures to light results in the rapid production of tablets.

Further details about the technology are known to a person skilled in the art and can e.g. be found in Acosta-Velez et al. Bioengineering (Basel). 2017 Mar; 4(1): 11.

In binder jetting, also called powder bed and ink jet head 3D printing, a liquid binding agent is selectively deposited to join powders.

The object is defined through the use of computer-aided design (CAD) software and digitally sliced in detailed pieces of information that delineate each one of the layers to be printed through the process. After each layer deposition, a piston that supports the powder bed is lowered allowing a subsequent layer of powder to be spread and selectively bound. This process is repeated several times, stacking layers of solidified material until a predetermined 3D geometry is produced. Excess powder not bound is then removed exposing the final product, which can go through further processing to tune its final mechanical and physical properties. Further details about the technology are known to a person skilled in the art and can e.g. be found in Acosta-Velez GF, Wu BM. 3D Pharming: Direct Printing of Personalized Pharmaceutical Tablets. Polym Sci. 2016, 1:2.

In Vat polymerization, also called stereolithography, a liquid photopolymer is selectively cured by polymerization. Coherent light sources (usually lasers emitting in the UV-range) are used to induce polymerization and cross-linking of the initially liquid resin. The time necessary to produce one slice of the structure therefore depends on the speed with which the laser beam is scanned and on the illuminated area. The lateral position of the laser beam is usually controlled by a pair of mirrors within a galvanoscanner. As with most other 3D printing technologies, the process is executed in a layer by layer manner. The slice information is presented in the form of a set of coordinates, defining the tilt angle of the two mirrors, which guide the position of the laser beam along the plane. The fact that every pixel of the layer is irradiated sequentially would theoretically allow adjustment of exposure dose for every pixel separately, by controlling the laser intensity.

Further details about the technology are known to a person skilled in the art and can e.g. be found in Samuel Clark Ligon et al., Chem Rev. 2017 Aug 9; 117(15): 10212-10290.

In selective laser sintering, also called power-bed fusion, focused thermal energy is used to fuse materials by melting.

Selective laser sintering (SLS) 3-dimensional printing is currently used for industrial manufacturing of plastic, metallic and ceramic objects. SLS is a versatile and practical 3D printing technology which can be applied to the several fields of technology. Further details about the technology are known to a person skilled in the art and can e.g. be found in Fina et al. Int J Pharm. 2017 Aug 30; 529(1-2):285-293.

It has been found that a certain method for 3D printing is especially suitable for producing the 3D printed solid forms of a cell culture medium.

The process is a contactless laser printing process that allows the production of the solid forms in a flexible manner and in conformity with the high quality standards required for the production of cell culture media ingredients.

The process uses a composite layer that comprises a laser energy absorbing layer and a layer that contains at least one cell culture media ingredient. The solid cell culture media form is build up by subsequent transfer of ingredient containing layer to one another by means of a laser beam.

With the aid of laser beams of various wavelength, it is possible to print and assemble successively layer on layer. The printing and assembling are carried out through the action of laser energy a) on the laser energy absorbing material itself (intrinsic reaction to generate heat increase and volume increase due to carbonization, foaming) and b) on the medium containing at least one cell culture media ingredient, which is transferred from the composite layer onto the assembling platform. If a laser beam of suitable energy and wavelength (for example Nd:Yag laser) hits a laser energy absorbing material and this is coated with a layer that contains a cell culture media ingredient, the cell culture media ingredient containing layer is transferred (printed) to the mounting plate, where it may be fixed thereon (e.g. by vacuum). Repeating in this way, assembling of layer by layer to a 3D form (e.g. a tablet) is provided. The amount of laser energy absorbing material actually required for the printing and assembling depends on laser type, energy output, printing speed, layer thickness of laser energy absorbing layer, film material thickness and adhesion of cell culture media ingredient containing layer (and force to transfer), dwell time of assembling steps.

Disclosed is a process for the manufacture of a solid 3D printed cell culture media form comprising at least one cell culture media ingredient comprising the steps
(a) positioning a composite layer (3) comprising a laser energy absorbing layer (1) and a layer that contains at least one cell culture media ingredient (2) between a plate (4) that is permeable for a laser beam that can be activated by a source of laser energy (5) and a mounting plate (6) whereat the layer of the composite containing at least one cell culture media ingredient (2) is positioned opposite to the source of laser energy (5) and is facing to the mounting plate (6);
(b) lowering the mounting plate (6) to shape an interspace (8) between the composite layer (3) comprising a cell culture media ingredient containing layer (2) and the mounting plate (6);
(c) transferring by action of laser beam from the source of laser energy (5) the cell culture media ingredient containing layer (2) of the composite (3) onto the mounting plate (6);
(d) repeating steps (a), (b) and (c) as often as needed to build up the solid cell culture media form;
(e) removing the solid cell culture media form from the mounting plate.

In an alternative embodiment the mounting plate (6) comprises an area that is movable in vertical direction. When running the process in step (b) not the whole mounting plate is lowered but the movable area only. Accordingly, disclosed is also directed to a process for the manufacture of a solid cell culture media form comprising at least one cell culture media ingredient comprising the steps
(a) positioning a composite layer (3) comprising a laser energy absorbing layer (1) and a layer that contains at least one cell culture media ingredient (2) between between a plate (4) that is permeable for a laser beam that can be activated by a source of laser energy (5) and a mounting plate (6) comprising at least one area (7) that is movable in vertical direction (z axis) relative to the mounting plate whereat the layer of the composite containing at least one cell culture media ingredient (2) is positioned opposite to the source of laser energy (5) and is facing to the mounting plate (6);
(b) lowering the movable area (7) relative to the mounting plate (6) to shape an interspace (8') between the composite layer (3) comprising a cell culture media ingredient containing layer (2) and the movable area (7) of the mounting plate (6);
(c) transferring by action of laser beam from the source of laser energy (5) the cell culture media ingredient containing layer (2) of the composite (3) into the interspace (8') shaped by the movable area (7) that was lowered vertically relative to the mounting plate (6);
(d) repeating steps (a), (b) and (c) as often as needed to build up the solid cell culture media form;
(e) removing the solid cell culture media form from the mounting plate.

Advantageously the movable area (7) in the mounting plate (6) is raised to the same level relative to the upper side of the mounting plate before the solid cell culture media form is removed.

The term "composite layer" as used herein means a layer comprising at least two layers that are attached to one another, each of said layers being comprised of a different material having a different function and composition. The composite layer comprises at least a laser energy absorbing layer and a layer that contains at least one cell culture media ingredient. Examples of further layers that can be present as part of the composite layer include separation layer(s) and adhesive layer(s) as defined and/or exemplified in this patent application.

The term "laser energy absorbing layer" as used herein means a layer that contains laser energy absorbing material as defined and/or exemplified in this patent application. The laser energy absorbing layer may be one layer, wherein an laser energy absorbing material is imbedded and/or distributed over the whole layer but also an assembly of layers comprising a layer that contains laser energy absorbing material (1 ") that is covered on one or both sides with layer(s) (1') and/or (1‴) that do not contain laser energy absorbing material (support layers).

The plate that is permeable for a laser beam is in fixed position relative to the mounting plate during the transfer (step (c)) and must have a sufficient mechanical strength to provide the back power needed for the transfer of the cell culture media ingredient containing layer in vertical direction relative to the surface of the laser permeable plate (downwards) to the mounting plate upon volume expansion of the composite layer that is triggered by the laser beam. The plate can consist of any material that is permeable for the laser beam and that has sufficient mechanical strength to provide the back power that is necessary for the transfer step. Suitable materials include glass such, for example, as quartz glass or borosilicate glass.

As used herein, "a" or "an" shall mean one or more. As used herein when used in conjunction with the word "comprising," the words "a" or "an" mean one or more than one. As used herein "another" means at least a second or more. Furthermore, unless otherwise required by context, singular terms include pluralities and plural terms include the singular.

As used herein, "about" refers to a numeric value, including, for example, whole numbers, fractions, and percentages, whether or not explicitly indicated. The term "about" generally refers to a range of numerical values (e.g., +/- 1-3% of the recited value) that one of ordinary skill in the art would consider equivalent to the recited value (e.g., having the same function or result). In some instances, the term "about" may include numerical values that are rounded to the nearest significant figure.

The shape of the solid cell culture media form can be easily determined by simply controlling the irradiation area of the laser beam. The irradiation area, i.e. the area of the composite that is activated by the laser beam, defines the area of the cell culture media ingredient containing layer that is transferred by laser activation. By controlling the shape of the irradiation area cell culture media ingredient containing layer any desired shape, such as rectangular, quadratic, cruciform, circular or oval, can be transferred. By assembling cell culture media ingredient containing layers with different shapes solid forms of any three-dimensional shape can be easily obtained. The process provides wide flexibility with respect to the shape of the solid form. Advantageously the shape of the solid form can be easily adapted to various specific demands and, in addition, allows new shapes that cannot be made available by conventional tablet manufacturing processes.

The process uses layers containing the cell culture media ingredient. As the cell culture media ingredient is embedded in the layer it is not necessary to handle the pure cell culture media ingredient so that the problems associated with such handling are avoided. The cell culture media ingredient containing layers are simply attached to each another so that dust formation and contamination of working environment that would require protective measures such as enclosed housing and extensive cleaning operations is avoided.

Further, solid forms with different dosages and/or different cell culture media ingredients can be manufactured in an easy manner. For example solid forms with different dosages but the same cell culture media ingredient can be manufactured by simply controlling the number of cell culture media ingredient containing layers that are attached to each other. Solid forms with the same cell culture media ingredient but different release properties such as an administration form wherein a part of the cell culture media ingredient is released in an immediate release manner and another part of is released in a sustained release manner can be manufactured by assembling cell culture media ingredient containing layers having immediate release properties and cell culture media ingredient containing layers having sustained release properties. In a similar manner solid forms with different cell culture media ingredients can be provided by assembling cell culture media ingredient containing layers wherein the different cell culture media ingredients are present as a mixture in the cell culture media ingredient containing layer and/or wherein the different cell culture media ingredients are present in different cell culture media ingredient containing layers. The latter is preferred if the cell culture media ingredients are incompatible to each other.

A switch from manufacturing of a solid form with a specific cell culture media ingredient to a different solid form with a different cell culture media ingredient can be performed by simply changing the composite layer comprising a layer that comprises one cell culture media ingredient to another composite layer that comprises another cell culture media ingredient without additional setup times. Solid forms, wherein one form contains the same cell culture media ingredient with different release properties (such as immediate release plus sustained release) or wherein different cell culture media ingredients are present, that are incompatible to each other, can also be easily manufactured using the by subsequently using different composite layers (having different cell culture media ingredients and/or cell culture media ingredient releasing characteristics) in the manufacture of that solid form. The process provides a maximum of flexibility and enables fast and easy operation without the need for time consuming cleaning operations and, therefore, is also suitable for the manufacture for specifically composed solid cell culture media forms which are adapted for a specific purpose in a decentralized manner.

According to a preferred embodiment the solid form is fixed during assembling at the mounting plate or the movable area by a vacuum. Therefore, disclosed is also a process that is characterized in that a vacuum is applied via a vacuum chuck (9) to hold the solid form on the mounting plate (6) or its movable area (7) during its assembling.

The composite layer used in the process can be a sheet or a tape. A tape is preferred as it can be easily handled and as it allows an easy positioning of it (step (a)) by using a roll to roll transport mechanism. Accordingly, disclosed is also a process that is characterized in that the composite layer (3) is provided as a tape (3') and that the positioning of the composite layer (3) in step (a) is achieved by roll (11) to roll (11 ') transport.

Advantageously the composite layer and the roll (11) to roll (11 ') mechanism for its transport is integrated in a cassette (16) which allows easy handling of the composite layer and its use in the process. Accordingly, disclosed is also a cassette (16) having a roll (11) to roll (11') transport mechanism wherein such roll (11) to roll (11') transport mechanism is equipped with the composite layer (3). Preferably the cassette comprises positioner rolls (4'), (4") which are moveable in up and down in vertical direction (z axis). This allows pressing / depressing of the composite layer (3) onto the mounting plate (6) depending from its status of operations.

In a further preferred embodiment the mounting plate is covered by a protection tape, which after completion of the manufacture of the solid form is moved along the mounting plate to (x-axis) provide an empty place for assembling a new solid form. Therefore, further disclosed is a process that is characterized in that the mounting plate is covered by a protection tape (10) and that such protection tape (10) is moved after completion of the solid form along the mounting plate (6) (x-axis) to provide an empty place for assembling a new solid form. Advantageously the protection tape can be easily replaced (exchanged) against a new one to avoid cross-contamination of materials, especially of the cell culture media ingredients, when the manufacturing process is changed from one solid form to another containing different cell culture media ingredient(s) and/or auxiliaries.

The protection tape can be made of any material that can be manufactured as tape and that provides protection of mounting plate against contamination with material from the composite layer, especially the cell culture media ingredient containing layer. Preferably, the protection material is permeable to air so that a solid form placed on top of it can be fixed by applying a vacuum through the chuck below of it. A suitable material for a protection tape is virgin paper. If a protection tape is used as described above no cleaning operations are needed when switching the process of the present invention from the manufacturing of a solid form to another solid form that contains different cell culture media ingredient(s).

According to a preferred embodiment, the protection tape is moved by using a roll to roll transport mechanism. Therefore, disclosed is also a process that is characterized in that the protection tape (10) is moved by roll (12) to roll (12') transport.

The process requires a composite layer that comprises a laser energy absorbing layer and a layer that contains at least one cell culture media ingredient. Accordingly, disclosed is also a composite layer that is usable for the process comprising a laser energy absorbing layer (1) and a layer that contains at least one cell culture media ingredient (2).

According to an embodiment the laser energy absorbing layer of the composite layer comprises a laser energy absorbing material that is covered on one or both sides with support layer(s) of a plastic material. The plastic material isolates the laser energy absorbing material from the environment and the cell culture media ingredient containing layer and prevents contamination especially of the ingredient containing layer and the assembled solid cell culture media form. Therefore, disclosed is further a composite layer which is characterized in that the laser energy absorbing layer (1) comprises a layer comprising a laser energy absorbing material (1") that is covered on one or both sides with support layers (1 '), (1‴) of a plastic material. In such embodiment support layers (1') and/or (1‴) and the layer containing the laser energy absorbing material (1") are bonded to one another as a unit. The support layers can be made of plastic material, wherein the material and/or thickness of layer (1') can be the same or different to layer (1‴).

In an alternative embodiment the laser energy absorbing material is imbedded in the plastic material and distributed over the whole energy absorbing layer. Contamination of environment and the cell culture media ingredient layer by the laser energy absorbing material is prevented by its embedment in the plastic material. Thus, further disclosed is a composite layer that is characterized in that the energy absorbing layer (1) consists of one layer, wherein a laser energy absorbing material is distributed within a plastic material.

Plastic material that is suitable for covering and/or embedding of the laser energy absorbing material that is present in the laser energy absorbing layer comprises polymers from the group of polyethylene (PE), polypropylene (PP), polyvinylchloride (PVC), polystyrol (PS), polytetrafluorethylene (PTFE), poly(methyl methacrylate) (PMMA), polyacrylnitril (PAN), polyacrylamid (PAA), polyamide (PA), aramide (polyaramide), (PPTA, Kevlar^{®}, Twaron^{®}), poly(m-phenylen terephthalamid) (PMPI, Nomex^{®}, Teijinconex^{®}), polyketons like polyetherketon (PEK), polyethylene terephthalate (PET, PETE), polycarbonate (PC), polyethylenglycol (PEG), polyurethane (PU), Kapton K and Kapton HN is poly (4,4'-oxydiphenylene-pyromellitimide), Poly(organo)siloxane, Melamine-resin (MF). Accordingly, as well disclosed is a composite layer that is characterized in that the plastic material is selected from the group of polymers from the group of polyethylene (PE), polypropylene (PP), polyvinylchloride (PVC), polystyrol (PS), polytetrafluorethylene (PTFE), poly(methyl methacrylate) (PMMA), polyacrylnitril (PAN), polyacrylamid (PAA), polyamide (PA), aramide (polyaramide), (PPTA, Kevlar^{®}, Twaron^{®}), poly(m-phenylen terephthalamid) (PMPI, Nomex^{®}, Teijinconex^{®}), polyketons like polyetherketon (PEK), polyethylene terephthalate (PET, PETE), polycarbonate (PC), polyethylenglycol (PEG), polyurethane (PU), Kapton K and Kapton HN is poly (4,4'-oxydiphenylene-pyromellitimide), Poly(organo)siloxane, Melamine-resin (MF).

The plastic material can be processed to layers using the methods known in the art such as, for example, by polymer extrusion, casting, calendaring and blow molding. Using such methods layers without laser energy absorbing material (support layers) and as well as the layer that contain laser energy absorbing material can be prepared and made available.

The term "laser energy absorbing material" as used herein means any material that absorbs laser light and converts it to some extend to heat. In principle, any laser energy absorbing material can be used. Laser energy absorbing materials that are especially suitable are indium oxide, indium tin oxide (ITO), antimon tin oxide (ATO), antimon oxide, tin oxide, zinc oxide, aluminium zinc oxide (AZO), a mixture of metal oxides, zinc sulfide, tin sulfide, carbon black, graphite, metal oxides, silicates, metal oxide coated mica or SiO2 flakes, a conductive pigment, sulfides, phosphates, BiOCI, anthracene, perylenes, rylenes, pentaerythritol or a mixture of two or more materials thereof. Therefore, disclosed is also a composite layer that is characterized in that the laser energy absorbing material is indium oxide, indium tin oxide (ITO), antimon tin oxide (ATO), antimon oxide, tin oxide, zinc oxide, aluminium zinc oxide (AZO), a mixture of metal oxides, zinc sulfide, tin sulfide, carbon black, graphite, metal oxides, silicates, metal oxide coated mica or SiO2 flakes, a conductive pigment, sulfides, phosphates, BiOCI, anthracene, perylenes, rylenes, pentaerythritol or a mixture of two or more materials thereof.

The laser energy absorbing material can be present in the laser energy absorbing layer in any particle size that is processible and that provides heat generation and distribution suitable for running the process. According to a preferred embodiment of the invention the composite layer is characterized in that the laser energy absorbing material present in the energy absorbing layer has a mean particle diameter from about 50nm to about 150nm.

The laser energy absorbing material can be present in the laser energy absorbing layer in any quantity that that is sufficient to provide the heat in an amount that is suitable for running the process. According to a preferred embodiment of the invention the composite layer is characterized in that the laser energy absorbing layer (1) comprises 0.01-20% by weight of laser energy absorbing material.

The process is based on a volume expansion of the laser energy absorbing layer and heat generation of the laser energy absorbing material as a result of activation of a laser beam, that both lead to the transfer of the cell culture media ingredient containing layer to the mounting plate or the movable area of the mounting plate. Volume expansion arises from foaming of the plastic material that is present in the laser energy absorbing layer and that is induced from heat and gas formation, especially carbonization (CO₂ formation) in the plastic material, and freezing of the generated foam upon subsequent cooling. Volume increase can be facilitated by the presence of copolymers of ethylene/ethylene acrylate, epoxy resins, polyesters, polyisobutylene, polyamides, polystyrene, acrylic polymers, polyamides, polyimides, melamine, urethane, benzoguanine and phenolic resins, silicone resins, micronized cellulose, fluorinated polymers (PTFE, PVDF inter alia) and micronized wax as filler or mixtures thereof which decompose and gives volume increase due to foaming, gas release and freezing. Accordingly, further disclosed is a composite layer that is characterized in that laser energy absorbing layer (1) contains copolymers of ethylene/ethylene acrylate, epoxy resins, polyesters, polyisobutylene, polyamides, polystyrene, acrylic polymers, polyamides, polyimides, melamine, urethane, benzoguanine and phenolic resins, silicone resins, micronized cellulose, fluorinated polymers (PTFE, PVDF inter alia) and micronized wax as filler or mixtures thereof.

The polymers for facilitating volume increase are admixed to the plastic material for embedding the laser energy absorbing material. They can be dissolved, for example by melt extrusion, in the plastic material to form a homogeneous material with the plastic material for embedding the laser energy absorbing material or admixed and maintained as particles within the such plastic material. If admixed and maintained as particles the polymers for facilitating volume increase preferably have mean particle sizes in the range from about10 nm to about 20 µm.

Although the plastic material present in the energy absorbing layer isolates and/or imbeds the laser energy absorbing material from the cell culture media ingredient containing layer and prevents it from contamination with the laser energy absorbing material it can be desirable to further separate the energy absorbing layer from the cell culture media ingredient containing layer. Beside additional prevention of the cell culture media ingredient containing layer from contamination a layer separating the energy absorbing layer from the cell culture media ingredient containing layer may improve the properties of the composite layer that are required for its use in the process. For example it may improve detachment characteristics of the cell culture media ingredient containing layer from the composite layer at the transferring step (step (c)). Therefore, disclosed is also a composite layer that is characterized in that it comprises a separation layer (13), which is located between the energy absorbing layer (1) and the layer that contains at least one cell culture media ingredient (2).

The separation layer can be made from any material that can be processed to a layer and can be attached to the laser energy absorbing layer and the cell culture media ingredient containing layer and that provides the required properties such as suitable detachment properties.

Suitable materials comprises saccharides, like disaccharides such as sucrose or lactose, polysaccharides such as starch, cellulose or derivatives thereof, modified celluloses such as microcrystalline cellulose and cellulose ethers, such as hydroxypropyl cellulose (HPC), croscarmellose sodium, sugar alcohols such as xylitol, sorbitol or maltitol, glucose, proteins such as gelatin, synthetic polymers such as polyvinylpyrrolidone (PVP), cross linked polyvinyl N-pyrrolidone or polyethylene glycol (PEG), poloxamer, Tragacanth, Gummi Arabicum, a low melting waxes such as beeswax, candelilla wax, carnauba wax, ceresine wax, microcrystalline wax, ozokerite wax, magnesium stearate, paraffin wax and combination thereof, preferred are disaccharides such as sucrose or lactose, polysaccharides such as starch, cellulose, sugar alcohols such as xylitol, sorbitol or maltitol, Gummi Arabicum, paraffin wax and combinations thereof, especially preferred are sugar alcohols such as xylitol, sorbitol or maltitol and paraffin wax and combinations thereof. Therefore, disclosed is also a composite layer that is characterized in that the separation layer (13) comprises at least a saccharide, which can be a disaccharide such as sucrose or lactose, a polysaccharide such as starch, cellulose or a derivative thereof, a modified cellulose such as a microcrystalline cellulose or a cellulose ether, such as hydroxypropyl cellulose (HPC), croscarmellose sodium, a sugar alcohol such as xylitol, sorbitol or maltitol, glucose, a protein such as gelatin, a synthetic polymer such as polyvinylpyrrolidone (PVP), cross linked polyvinyl N-pyrrolidone or polyethylene glycol (PEG), poloxamer, Tragacanth, Gummi Arabicum, a low melting wax such as beeswax, candelilla wax, carnauba wax, ceresine wax, microcrystalline wax, magnesium stearate, ozokerite wax or paraffin wax and combinations thereof. The separation layer can have a thickness from 2 µm to 150 µm, preferably from 5 µm to 100 µm, more preferably from 10 µm to 30 µm and most preferably from 10 µm to 20 µm, especially of about 17 µm.

Depending from the requirements of the cell culture media ingredient present in the cell culture media ingredient containing layer and the desired release properties of the solid cell culture media form it may be difficult in some cases to provide an cell culture media ingredient containing layer that has the physicochemical characteristics, especially the adhesive properties, that are required for sufficient attachment of the cell culture media ingredient containing layers one to another during assembling in the process. In such cases an additional layer with adhesive properties, i.e. an adhesion layer, can be placed on the cell culture media ingredient containing layer located outward and opposite to the energy absorbing layer of the composite. Accordingly, disclosed is as well a composite layer that is characterized in that it comprises an adhesion layer (14) which is located outward and opposite to the energy absorbing layer (1). In the transfer step (step (c)) the adhesion layer is detached from the laser energy absorbing layer and transferred to the mounting plate or the movable area on it together with the cell culture media ingredient containing layer.

Materials that can be used for an adhesion layer can be any material that do not have adverse effect on the cell culture and that provide the required adhesive properties. Materials that can be used for the adhesion layer comprise methyl cellulose, liquid glucose, tragacanth, ethyl cellulose, gelatin, hydroxy propyl methyl cellulose (HPMC), starch paste, hydroxy propyl cellulose, pregelanized starch, sodium carboxy methyl cellulose, algenic acid, polyvinyl pyrollidone (PVP), cellulose, gummi arabicum, polyethylene glycol (PEG) and combinations thereof. Preferred are methyl cellulose, liquid glucose, tragacanth, gelatin, starch paste, sodium carboxy methyl cellulose, algenic acid, cellulose, gummi arabicum, and combinations thereof, especially preferred are gelatin, starch paste and algenic acid. Therefore, disclosed is further a composite layer that is characterized in that the adhesion layer (14) comprises methyl cellulose, liquid glucose, tragacanth, ethyl cellulose, gelatin, hydroxy propyl methyl cellulose (HPMC), starch paste, hydroxy propyl cellulose, pregelanized starch, sodium carboxy methyl cellulose, algenic acid, polyvinyl pyrollidone (PVP), cellulose, gummi arabicum, polyethylene glycol (PEG) or combinations thereof.

The terms "layer containing at least one cell culture media ingredient" and "cell culture media ingredient containing layer" as used herein are used synonymous and both means a layer that contains at least one cell culture media ingredient. Beside the cell culture media ingredient suitable auxiliaries might be present in the layer to provide a matrix for the distribution of the cell culture media ingredient and to provide the framework and properties that are required for the manufacture of the cell culture media ingredient containing layer, its use in the process and the stability and release properties of the cell culture media ingredient after application of the solid form to the solvent in case the solid form is a base medium, the liquid cell culture base medium or the cell culture in case the solid form is a media supplement. The layer containing at least one cell culture media ingredient usually is a continuous uninterrupted layer. Alternatively, however, the layer containing at least one cell culture media ingredient also encompass a layer, wherein the layer is divided in multitude of pieces with defined geometry (2'), (19e) - (19i), (20a) - (20h), (e.g. squares, rectangles, hexagons etc.) that are separated from each other by another layer, such as, for example, the laser energy absorbing layer (1) or a support layer (1‴). A cell culture media ingredient containing layer, wherein the layer is divided in a multitude of pieces can be provided by first providing a laser energy absorbing layer (1) with engraved cavities (1ʺʺ) having a defined geometry for shaping the geometry of the pieces and subsequent filling the cavities in the energy absorbing layer with the material constituting the cell culture media ingredient containing layer, for example by using a doctor blade. The cell culture media ingredient containing layer can have a thickness from 5 µm to 1000 µm, preferably from 10 µm to 500 µm, more preferably from 30 µm to 200 µm and most preferably from 50 µm to 100 µm, especially of about 70 µm.

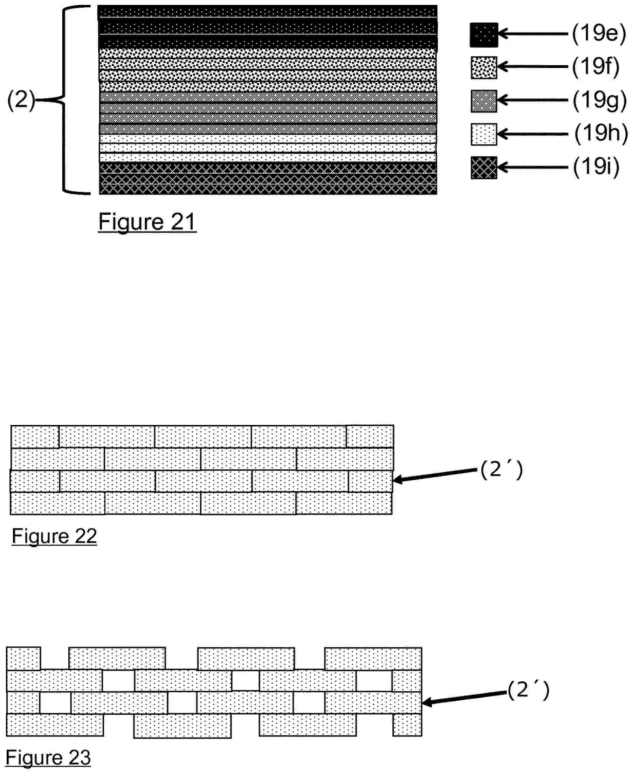

If a composite layer (3) that comprises a cell culture media ingredient containing layer that is formed as a continuous uninterrupted layer (2) is used in the process solid forms are manufactured that consist of a continuous uniform body. If a composite layer is used, wherein the layer containing at least one cell culture media ingredient is divided in a multitude of pieces solid forms can be manufactured that either have a closed structure or have an open, porous structure as exemplified in Figures 22 and 23 respectively. Dissolution and release of cell culture media ingredient from a solid form depends, i.a. from its geometry and the surface that is exposed to the environment after application. Therefore, changing the shape and surface area of the solid form that is exposed to dissolution after its application by using composite layers that comprise a layer containing at least one cell culture media ingredient that is divided in a multitude of pieces and by variation of their arrangement during assembling of the cell culture media ingredient containing layers to the solid form offers a good opportunity to adapt the dissolution / release properties of the solid form to the needs. Therefore, disclosed is also a composite layer that is characterized in that the layer containing at least one cell culture media ingredient that is divided in a multitude of pieces (2'). The term "a multitude of" or "multiple" as used herein means at least 9 pieces per cm². According to suitable embodiments the layer containing at least one cell culture media ingredient is divided in into 10-20, 20-1000 or 1000-10000 pieces per cm².

According to a further appropriate embodiment the layer containing at least one cell culture media ingredient that is divided in a multitude of pieces (2') is patterned with a square shape and/or rectangular shape and/or round shape and/or oval shape.

According to an further appropriate embodiment the layer containing at least one cell culture media ingredient that is divided in a multitude of pieces (2') consists of multiple squares with edge length of 10µm up to 2500µm, or multiple round dots with diameter of 10 µm up to 3000 µm and gap to adjacent squares of 10 µm up to 2000 µm.

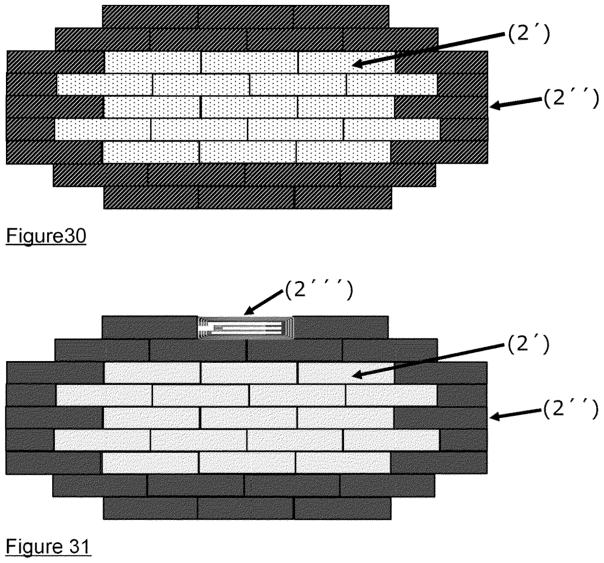

The process can also be used for the manufacture of a solid cell culture media form that comprises a cell culture media ingredient containing layer that is surrounded by a layer that does not contain a cell culture media ingredient thereby forming a core-shell structure. In such embodiment the cell culture media ingredient containing layer is divided in a multitude of pieces (2') and the layer that does not contain a cell culture media ingredient is divided in a multitude of pieces (2") as well. In alternative embodiments the shell contains the same cell culture media ingredient in a different amount, such as, for example in a higher dosage that is rapidly released after administration of the solid form due to the first dissolution of the (outer) shell that builds up an initial high level in the liquid cell culture medium prior to release of the same cell culture media ingredient from the core, or a different cell culture media ingredient, which after administration of the solid form due to the first dissolution of the (outer) shell is released first and prior to the release of the (different) cell culture media ingredient present in the core thereby achieving subsequent and time controlled release of different cell culture media ingredients. Embodiments of a solid form having such a core shell structure are exemplified in Figures 30 to 33.

Dissolution and release profile of cell culture media ingredients of the core-shell structure can be varied over a wide range depending on the demands. For example, adjustment of the dissolution profile can be performed by careful selection of the material that builds up the shell of the system (e.g. enteric coating such as Eudragit L 100-55 or polyvinyl acetate phthalate or non-enteric coating such as hydroxyethyl cellulose) and/or the thickness of the shell material.

The process allows also the manufacture of more complex systems such as core-shell structures with more than one shells wherein the core-shell structure is surrounded by one or more additional shells attached to each other. Depending on the demands the inner shells but also the outer shell may contain one or more cell culture media ingredients whereby the cell culture media ingredient may be the same or different ones. By applying this principle various solid forms can be provided from which the cell culture media ingredient/s is/are released in a predetermined manner according the specific demands.

This is especially suitable for fed batch cell culture in which presently complicated feeding strategies necessitate the addition of different media supplements (in fed batch also called feed media) at times points of time during the process. This can be simplified by using solid form which have e.g. core-shell structures causing direct and/or and sustained release of selected cell culture media ingredients.

The process also allows the exact placement of a communication device within the solid form. A communication device may be used in the solid cell culture media form to give information on the condition of the composite layer in the cell culture. For example information on the time when the composite layer disintegrates and the cell culture media ingredient is released may be given. With this information e.g. the feed strategy and the composition of the solid form to be added in a fed batch process can be further optimized. Examples of communication devices that can be placed in the solid form are a RFID (Radio Frequency Identification) tag, an electromagnetic signaling device, a magnetic device, an infrared emitting device or an ultrasonic device. Preferably the communication device is a RFID tag.

Depending from the information to be provided the communication device can be placed at any position of each layer of the solid form. In the following some embodiments are shown, wherein one or more RFID tag(s) (2‴) is/are placed in a solid form that is arranged as core shell system (Figures 31 to 33). Of course RFID tags can be placed also to any other place within the respective arrangement.

Suitable auxiliaries that can be used as material for the cell culture media ingredient containing layer are all auxiliaries that are known in the art that provide a structure and properties that are necessary and that are suitable as auxiliaries for e.g. tableting. Such auxiliaries include, for example, matrix building polymers, such as, for example, polyvinyl pyrrolidone or hydroxypropyl cellulose, disintegrants, such as, for example, carboxymethylcellulose sodium, croscarmellose sodium, surfactants, such as, for example, benzalkonium chloride or cetrimide, adhesives, such as, for example, polymethacrylates, tackifiers, such as, for example, poly (β-pinene), etc. As an example, the material for the cell culture media ingredient containing layer can be a mixture of a high concentrated mixture of cell culture media ingredient, binders, fillers and adhesives from the list: Talkum, Magnesium carbonate, Methyl Cellulose, Liquid Glucose, Tragacanth, Ethyl Cellulose, Gelatin, Hydroxy Propyl Methyl Cellulose (HPMC), Starch Paste, Hydroxy Propyl Cellulose, Pregelanized Starch, Sodium Carboxy Methyl Cellulose, Algenic Acid, Polyvinyl Pyrollidone (PVP), Cellulose, Gummi Arabicum, Polyethylene Glycol (PEG), poloxamer. In a preferred embodiment, no additional auxiliaries are necessary as the cell culture media ingredients have all necessary properties. This is for example the case if one of the cell culture media ingredients is poloxamer.

According to an appropriate embodiment the layer containing at least one cell culture media ingredient that is present in the composite layer comprises the cell culture media ingredient in an amount from 0.1% to 100% by weight. Preferably, the solid form comprises as little auxiliaries as possible or no auxiliaries.

According to an appropriate embodiment the layer containing at least one cell culture media ingredient that is present in the composite layer comprises no more than 25% moisture.

As described above the composite layer is preferably used in the process in the form of a tape, which can be easily handled in the process using a roll to roll transport system. To improve and simplify the handling of the composite layer in the process the composite layer can be provided in recoiled form in a roll dispenser. If recoiled in a dispenser the composite layer is protected against physical damage and other harmful environmental influences and can be easily transported and stored. When the composite layer is needed to be used in the process it can easily be made available, for example by using a simple docking mechanism that connects the dispenser with the manufacturing equipment. Accordingly, disclosed is also the composite layer that is characterized in that it is provided in a recoiled form in a roll dispenser (11").

Composite layer (3) can be prepared using a multiple process steps that includes mixing and coating steps and includes various techniques known in the art such as extrusion and/or lamination techniques.

For example, mixing of materials of that are contained in one layer (e.g. laser energy absorbing material and a plastic material or laser energy absorbing material, a plastic material and a polymer that facilitates volume increase upon activation by laser beam that constitutes the laser energy absorbing layer or the ingredients that are contained in the cell culture media ingredient containing layer, the adhesion layer or the separation layer) can be performed by using appropriate mills such as, for example, a high speed mixer or a roll mixer.

In principle composite layer is prepared by successively applying one layer to another layer until the final composite is built up. For example, a composite layer according to Figure 4 or 5 is manufactured by applying the cell culture media ingredient containing layer (1) to the laser energy absorbing layer (2) and a composite layer according to Figure 8 is prepared by first applying the separation layer (13) to the laser energy absorbing layer (2), then applying cell culture media ingredient containing layer (1) the separation layer and finally applying the adhesive layer (14).

The techniques that can be used for applying one layer to the other can be any method known in the art, such as, for example, coating techniques using a doctor blade, melt extrusion coating and various printing techniques, e.g. screen printing, stencil printing, silk-screen printing, pad printing, stamp printing, gravure printing, mikrojet-printing and ink-jet printing. Depending from the technique further steps can be necessary, such as a drying step after coating with a doctor blade or cooling after melt extrusion. Screen printing, silk-screen printing, pad printing, stamp printing, gravure printing, mikrojet-printing and ink-jet printing are preferred methods for applying one layer to another to form the composite layer (3).

Usually the manufacture of the composite starts with providing the laser energy absorbing layer (1). Such laser energy absorbing layer can be prepared by homogeneous distribution of the laser energy absorbing material (e.g. carbon black, ATO) in the plastic material and subsequent film manufacturing with extrusion (e.g. blown film extrusion). If the laser energy absorbing layer (1) contains one or two support layers (1') and/or (1 ") such layer(s) can be applied to it by lamination, e.g. by using a roll laminator.

In an example preparation of the composite layer (3) includes multiple process steps starting with the mixing process for the laser energy absorbing material. Homogeneous distribution during the mixing step of selected raw materials is achieved, for example, with high speed dissolver and/or 3 roll mill. Coating of the laser energy absorbing layer onto a polymer film with defined thickness is feasible with doctor blade technique or a printing process. A convection oven or belt furnace is used for drying of the coated substrates. The dry laser energy absorbing layer will be completely encapsulated due to lamination of a second polymer film on top. Another mixing step is used for the cell culture media ingredient vehicle with high speed dissolver and 3 roll mill. Coating of the cell culture media ingredient layer onto the composite layer (1) with defined thickness and pattern is feasible with a printing process (e.g. screen printing or stencil printing).

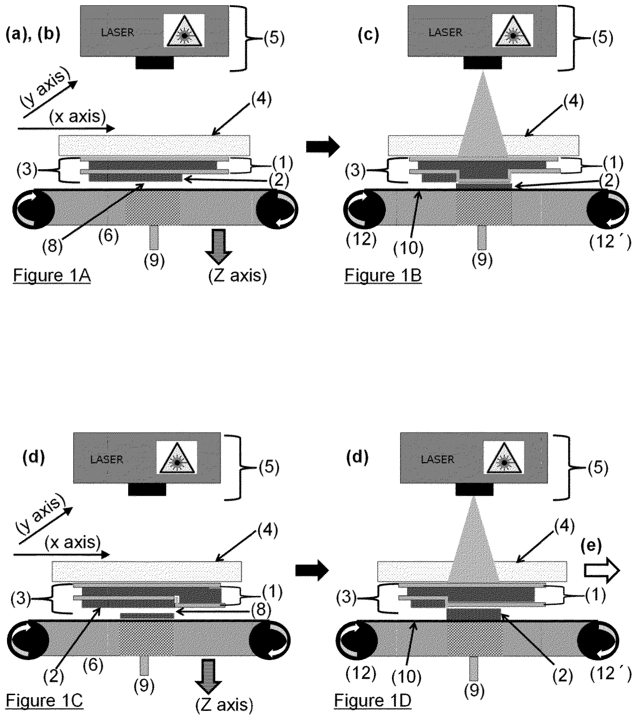

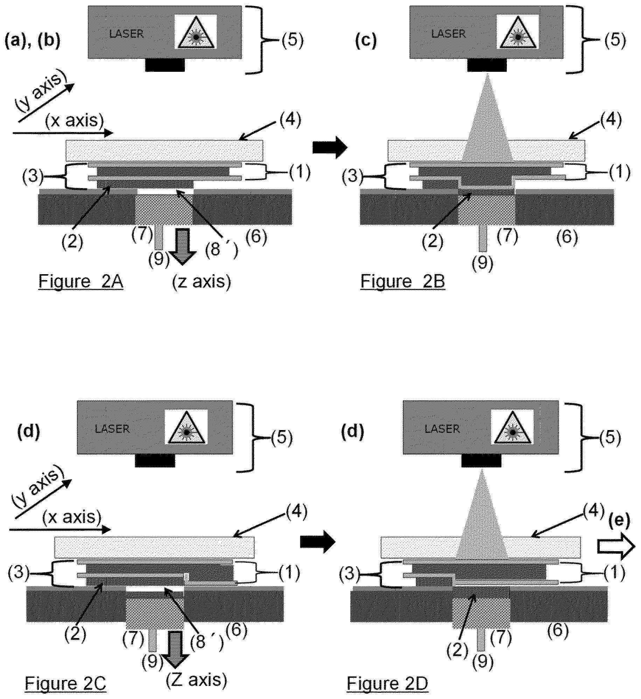

FIGS. 1 A to 1 D shows the configuration and the process steps (a), (b) and (c) of the process using a composite layer (3). The configuration differs from the configuration of FIG. 2 in that it does not comprise a movable area of the mounting plate (6). Instead the whole mounting plate can be moved down (in z-axis). Further it comprises a protection tape (10) that is arranged underneath the composite layer (3) and can be moved by a roll to roll system (12), (12').

The composite layer (3) is positioned onto the mounting plate (6) and fixed above the mounting plate (6) with a glass plate (4) (step (a)). The mounting plate (6) is moved down (z-direction) to provide a gap (8) having the same thickness as the cell culture media ingredient containing layer (FIG 1 A). The programmed laser (5) is activating the transfer step of the layer that contains at least one cell culture media ingredient (2) onto the mounting plate (6) (step (c)) (FIG 1 B). After transfer of the first layer that contains at least one cell culture media ingredient (2) onto the mounting plate (6) the composite layer (3) is moved along the x-axis and/or y-axis to provide new cell culture media ingredient containing layer above the (first) layer on the mounting plate (6) and steps (b) and (c) are repeated (FIGS 1 C and D). Positioning of new cell culture media ingredient containing composite layer (3) above the assembled layers on the mounting plate (step (a)) and steps (b) and (c) are repeated as often as needed to assemble the solid form. Adherence of the cell culture media ingredient containing layer(s) on the mounting plate (6) can be supported by applying a vacuum at the vacuum chuck (9). After complete assembly of the solid form it can be moved by moving the protection tape (10) along the x-axis by activating of the roll to roll system (12), (12') and is removed (step (e)).

FIGS. 2 A to 2 D show the configuration and the steps (a), (b) and (c) of the process using a composite layer (3). The composite layer (3) is positioned onto the movable area (7) of the mounting plate (6) and fixed above with a glass plate (4) (step (a)). An area of the mounting plate (6) is moved down to provide a gap (8') having the same thickness as the cell culture media ingredient containing layer (step (b)) (FIG 2 A). The programmed laser (5) is activating the transfer step of the layer that contains at least one cell culture media ingredient (2) onto the movable area (7) of the mounting plate (6) (step (c)) (FIG 2 B). After transfer of the first layer that contains at least one cell culture media ingredient (2) onto movable area (7) of the mounting plate (6) the composite layer (3) is moved along the x-axis and/or y-axis to provide new cell culture media ingredient containing layer above the (first) layer on movable area (7) of the mounting plate (6) and steps (b) and (c) are repeated (FIGS 2 C and D). Positioning of new cell culture media ingredient containing composite layer (3) above the assembled layers on the movable area (7) of the mounting plate (6) (step (a)) and steps (b) and (c) are repeated as often as needed to assemble the solid form. Adherence of the cell culture media ingredient containing layer(s) on movable area (7) of the mounting plate (6) can be supported by applying a vacuum at the vacuum chuck (9). After complete assembly the form is removed (step (e)). Advantageously the movable area (7) in the mounting plate (6) is raised to the same level relative to the upper side of the mounting plate before the solid form is removed.

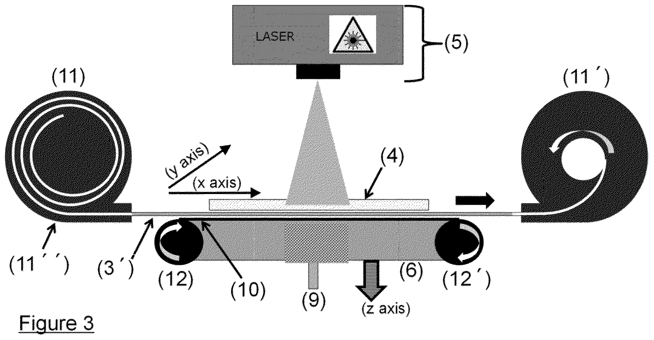

FIG. 3 shows an advantageous configuration that can be used, wherein with a composite layer (3) is provided as a tape (3') and transported by roll to roll dispensing system (11), (11'). The mounting plate (6) is movable in z-direction and sheeted by a protection tape (10), which is movable along x-axis by activation of another roll to roll system (12), (12'). The programmed laser (5) is activating the print step of the cell culture media ingredient onto the mounting plate (6). During assembling the solid form is fixed by a vacuum chuck (9).

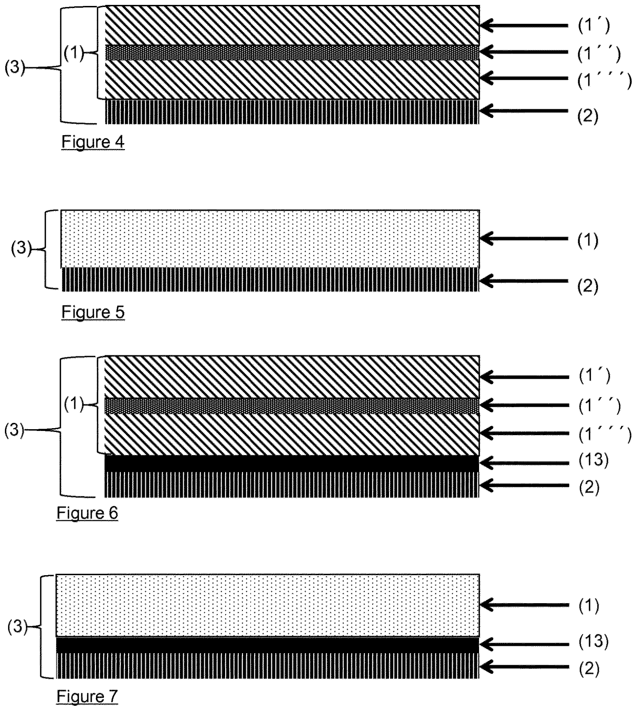

FIG. 4 shows a composite layer (3) consisting of a laser energy absorbing layer (1) and a layer that contains at least one cell culture media ingredient (2), wherein the laser energy absorbing layer (1) comprises a layer comprising the laser energy absorbing material (1") that is covered on both sides with support layers (1') and (1‴) that does not contain laser energy absorbing material and that are transparent and stable to laser light.

FIG. 5 shows a composite layer (3) consisting of an energy absorbing layer (1) that consists of one layer, which contains a laser energy absorbing material, and a layer that contains at least one cell culture media ingredient (2).

FIG. 6 shows a composite layer (3) as in FIG. 4, wherein a separation layer (13) is present between a support layer (1‴) and the layer that contains at least one cell culture media ingredient (2).

FIG. 7 shows a composite layer (3) as in FIG. 5, wherein a separation layer (13) is present between the laser energy absorbing layer (1) and the layer that contains at least one cell culture media ingredient (2).

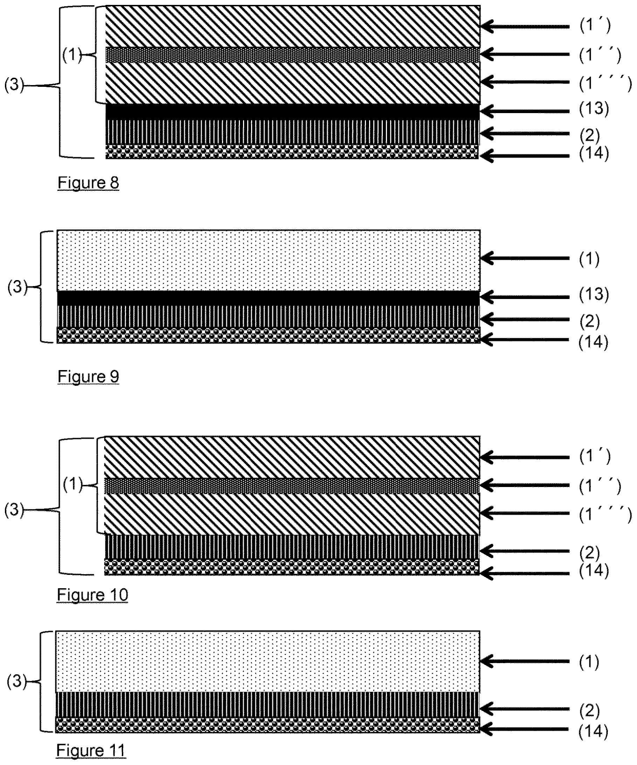

FIG. 8 shows a composite layer (3) as in FIGS. 6, wherein an adhesion layer (14) is present on the bottom side of the layer that contains at least one cell culture media ingredient (2).

FIG. 9 shows a composite layer (3) as in FIG. 7, wherein an adhesion layer (14) is present on the bottom side of the layer that contains at least one cell culture media ingredient (2).

FIG. 10 shows a composite layer (3) as in FIG. 4, wherein an adhesion layer (14) is present on the bottom side of the layer that contains at least one cell culture media ingredient (2).

FIG. 11 shows a composite layer (3) as in FIG. 5, wherein an adhesion layer (14) is present on the bottom side of the layer that contains at least one cell culture media ingredient (2).

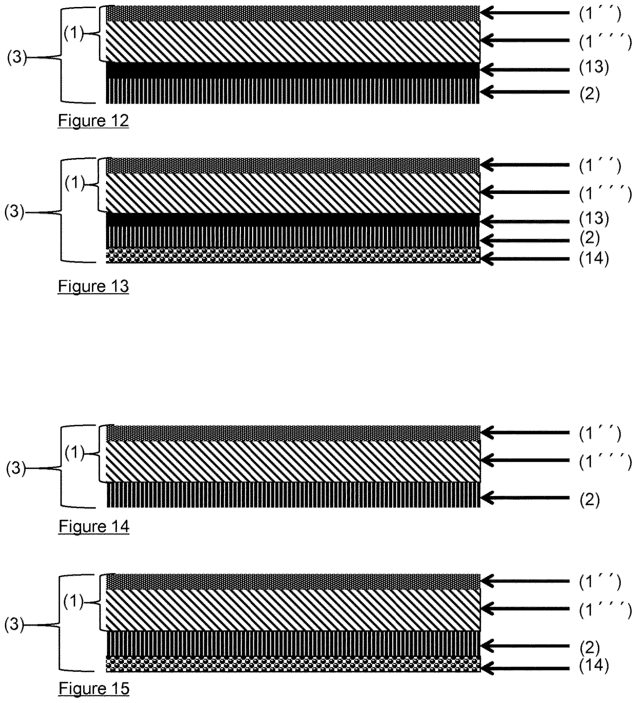

FIG. 12 shows a composite layer (3) consisting of a laser energy absorbing layer (1) consisting of a layer with a laser energy absorbing material (1") and a support layer (1‴), a separation layer (13) and a layer that contains at least one cell culture media ingredient (2).

FIG. 13 shows a composite layer (3) as in FIG. 12, wherein an adhesion layer (14) is present on the bottom side of the layer that contains at least one cell culture media ingredient (2).

FIG. 14 shows a composite layer (3) as in FIG. 12 but without a separation layer (13).

FIG. 15 shows a composite layer (3) as in FIG. 14, wherein an adhesion layer (14) is present on the bottom side of the layer that contains at least one cell culture media ingredient (2).

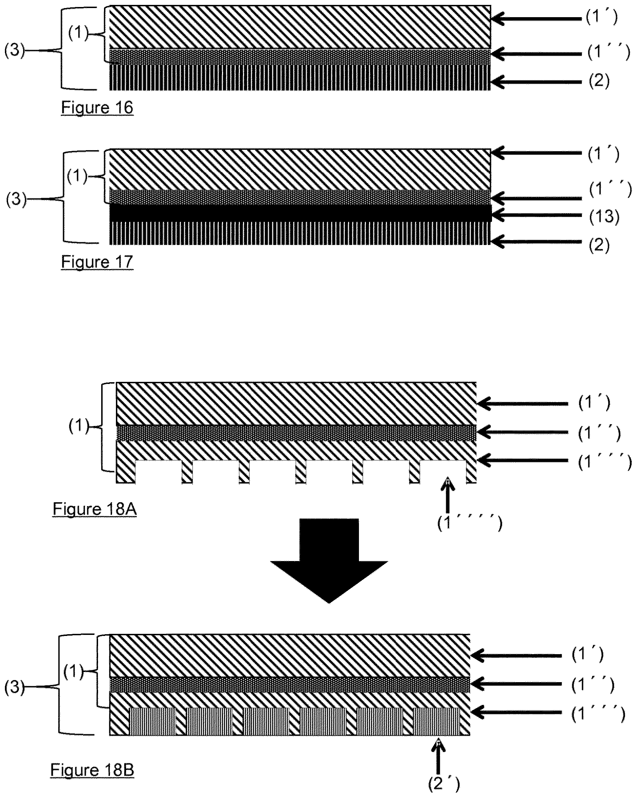

FIG. 16 shows a composite layer (3) consisting of a support layer (1') a layer comprising a laser energy absorbing material (1 ") and a layer that contains at least one cell culture media ingredient (2) at the bottom side.

FIG. 17 shows a composite layer (3) as in FIG. 16, wherein a separation layer (13) is present between the layer comprising the laser energy absorbing material (1") and the layer that contains at least one cell culture media ingredient (2).

FIG. 18 A shows an advanced variant of the composite layer comprising a laser energy absorbing layer (1) consisting of a support layer (1'), a layer comprising a laser energy absorbing material (1") and a support layer (1‴), wherein the support layer (1‴) has engraved cavities (1ʺʺ) that can be filled with material containing at least one cell culture media ingredient and that constitute the pieces (2') of the layer comprising at least one cell culture media ingredient.

FIG 18 B shows such composite layer (3), wherein the cavities (1ʺʺ) are filled with material containing at least one cell culture media ingredient and constitute the pieces (2') of the layer comprising at least one cell culture media ingredient. The layer containing at least one cell culture media ingredient is divided in a multitude of pieces (2'). Cavity size depth can be from 50 µm up to 500 µm (18 A). Cavity size diameter can be 50 µm up to 5 mm.

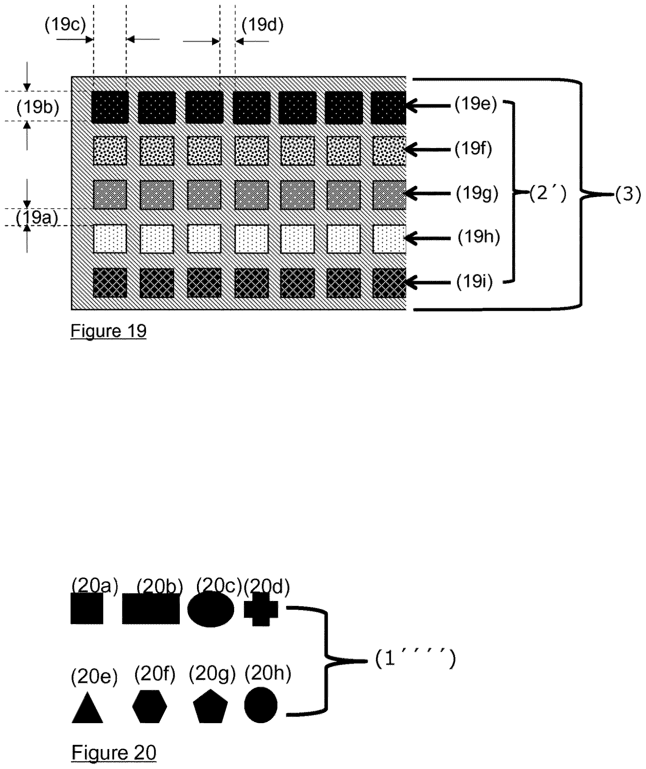

FIG. 19 shows the bottom side of a composite layer (3) with square sized cavities arranged in 5 different rows, wherein such cavities are filled or imprinted with material containing at least one cell culture media ingredient and constitute the pieces (2') of the layer comprising at least one cell culture media ingredient and wherein each of the different rows (19e), (19f), (19g), (19h) (19i) contains material with a cell culture media ingredient that is different from the others. Various cell culture media ingredients can be present in the layer containing at least one cell culture media ingredient that is divided in a multitude of pieces (2'). Gap between the cavities / pieces can be from about 50 µm to about 1 mm (19a, 19d). Side length of the cell culture media ingredient cavity or printed layer can be 50 µm up to 5 mm (19b, 19c).

FIG. 20 shows different options for shape of the cavities (1ʺʺ) engraved or imprinted in the support material (1‴) that can be filled with material containing at least one cell culture media ingredient, i.e. square (20a), rectangle (20b), oval (20c), cross (20d), triangle (20e), hexagon (20f), pentagon (20g), disk (20h).

FIG. 21 shows a sectional view of a solid form that is sequentially formed by using the process and the composite layer disclosed in FIG. 19.

FIG. 22 and 23 show cross sectional views of a solid form that can be prepared using the process and the composite layer shown in FIG. 18 B. The pieces of material comprising at least one cell culture media ingredient (2') that are present in one layer are in an offset position relative to the pieces of material containing at least one cell culture media ingredient (2') that are present in a layer on top of such layer.

FIG. 22 shows a solid form, wherein each piece of material comprising at least one cell culture media ingredient (2') that is present in a layer is arranged adjacent to one another thereby forming a closed structure of the solid form.

FIG. 23 shows a solid form, wherein each piece of material comprising at least one cell culture media ingredient (2') that is present in a layer is arranged apart to one another thereby forming an open structure of the solid form. Compared to the high density arrangement shown in FIG. 22 that provides a limited dissolution rate of cell culture media ingredient (2) the low density arrangement shown in FIG. 23 provides an enhanced dissolution rate of cell culture media ingredient (2).

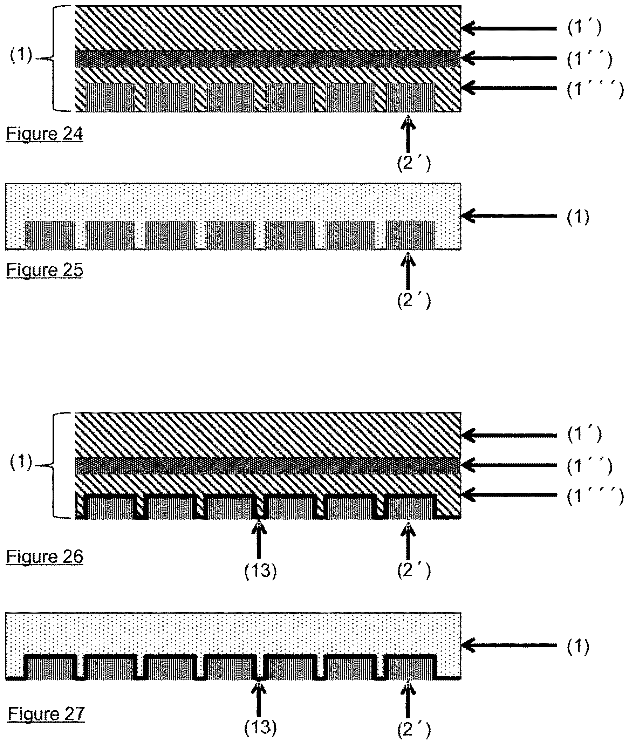

FIG. 24 shows a composite layer as in FIG. 4, wherein the support layer (1‴) is engraved and includes cavities filled with material comprising at least one cell culture media ingredient that constitute the pieces (2') of the layer comprising at least on cell culture media ingredient. The cavities are filled, for example, with compressed cell culture media ingredient, viscous- or even liquid cell culture media ingredient (option for closed cavities due to additional film layer).

FIG. 25 shows a composite layer as in FIG. 5, wherein the energy absorbing layer (1) has engraved cavities that are filled with material comprising at least one cell culture media ingredient that constitute the pieces (2') of the layer comprising at least on cell culture media ingredient.

FIG. 26 shows a composite layer as in FIG. 6, wherein the support layer (1‴) and the separation layer (13) have engraved cavities that are filled with material comprising at least one cell culture media ingredient that constitute the pieces (2') of the layer comprising at least one cell culture media ingredient.

FIG. 27 shows a composite layer as in FIG. 7, wherein the laser energy absorbing layer (1) and the separation layer (13) have engraved cavities that are filled with material comprising at least one cell culture media ingredient that constitute the pieces (2') of the layer comprising at least one cell culture media ingredient.

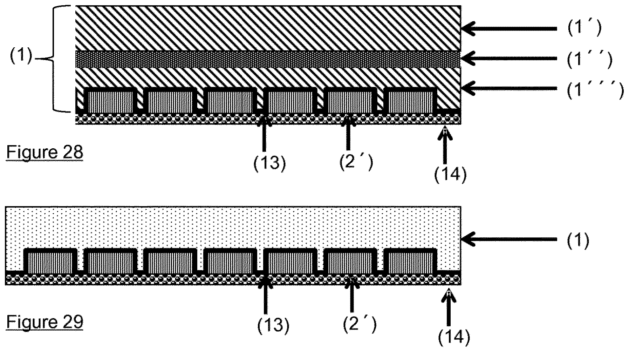

FIG. 28 shows a composite layer as in FIG. 26, wherein an adhesion layer (14) is attached on the bottom side of the cell culture media ingredient containing layer that is divided into pieces (2').

FIG. 29 shows a composite layer as in FIG. 27, wherein an adhesion layer (14) is attached on the bottom side of the cell culture media ingredient containing layer that is divided into pieces (2').

FIG. 30 shows a solid form which is arranged as core-shell system, wherein the core is made of a multitude of pieces that contain a cell culture media ingredient (2'), which is surrounded by a shell that is made of a multitude of pieces that does not contain a cell culture media ingredient (2"). Core and shell are arranged adjacent to one another thereby forming a closed structure of a solid form.

FIG. 31 shows a solid form as in FIG. 30, wherein a RFID tag (2‴) is placed in the shell made of a multitude of pieces that do not contain a cell culture media ingredient (2"). Due to the geometrical arrangement, upon dissolution of the solid form, the RFID tag (2‴) is released at first together with the dissolution of the shell which is followed by dissolution and release of cell culture media ingredient (2')

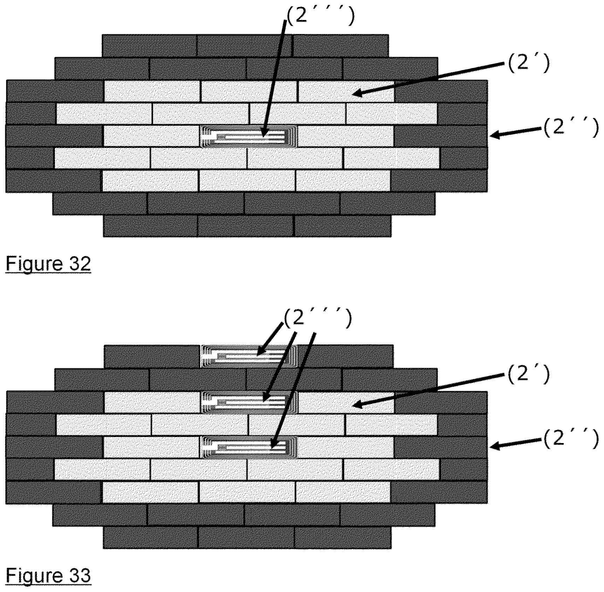

FIG. 32 shows a solid form as in FIG. 30, wherein a RFID tag (2‴) is placed in the core made of a multitude of pieces that contain a cell culture media ingredient (2'). Due to the geometrical arrangement, upon dissolution of the solid form, exposure and release of the RFID tag (2‴) and cell culture media ingredient (2') to the liquid is delayed as the shell surrounding the core is dissolved at first.

FIG. 33 shows a solid form as in FIG. 30 that contains 3 RFID tags (2‴) placed in the shell made of a multitude of pieces that does not contain a cell culture media ingredient (2"), in the outer surface and in the middle of the core made of a multitude of pieces that contain a cell culture media ingredient (2'). By integration of more than one RFID tags the investigation (tracking) period is increased and so that the behavior of the solid form can be monitored from the beginning of the dissolution until its final disintegration and/ or dissolution.

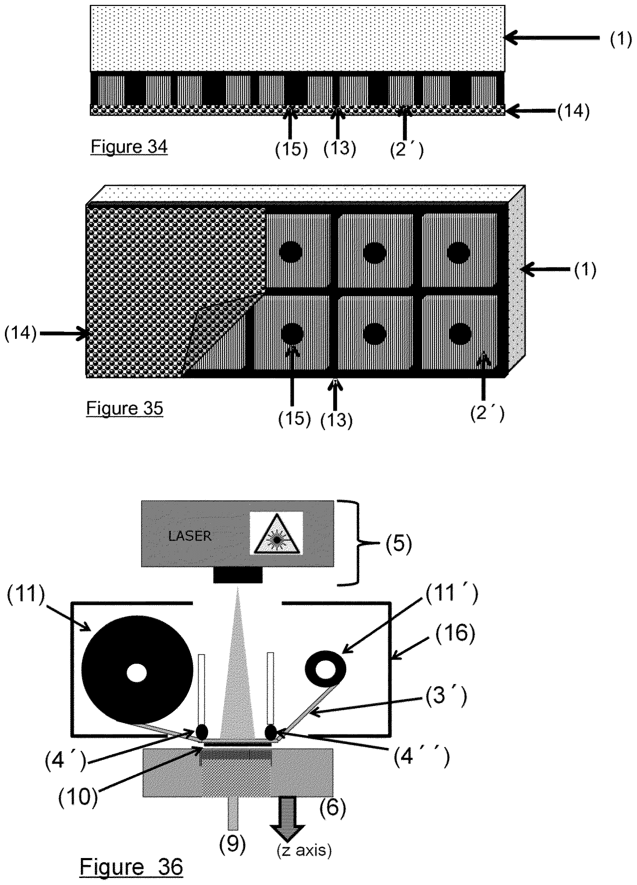

FIG. 34 shows a composite layer wherein a separation layer (13) is present between the laser energy absorbing layer (1) and the cell culture media ingredient containing layer that is divided into square shaped pieces (2') having a pinhole (15) in the center, wherein an adhesion layer (14) is attached on the bottom side of said cell culture media ingredient containing layer.

FIG. 35 shows the top view of a composite layer as in FIG. 34, which shows the same composite layer as cross-sectional view.

FIG. 36 shows an preferred configuration that can be used for the process, wherein with a composite layer (3) is provided as a tape (3') and transported by roll to roll dispensing system (11), (11') in a cassette box (16). The cassette system is changeable. Positioner rolls (4'), (4") is moveable in vertical direction thereby allowing pressing / depressing of the composite layer (3) onto the mounting plate (6) depending from its status of operations. The mounting plate (6) is movable in z-direction and sheeted by a protection tape (10). The laser (5) is activating the print step of the cell culture media ingredient onto the mounting plate (6). During assembling the solid form is fixed by a vacuum chuck (9).

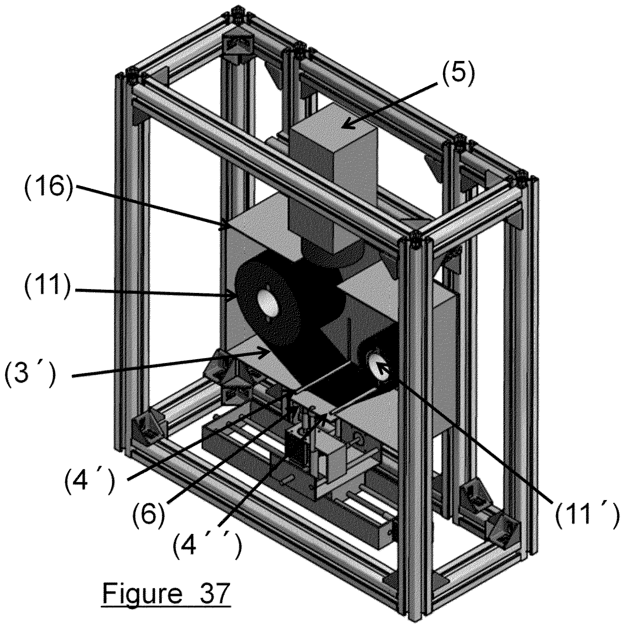

FIG. 37 shows the same configuration as shown in in FIG. 35 as a three-dimensional view.

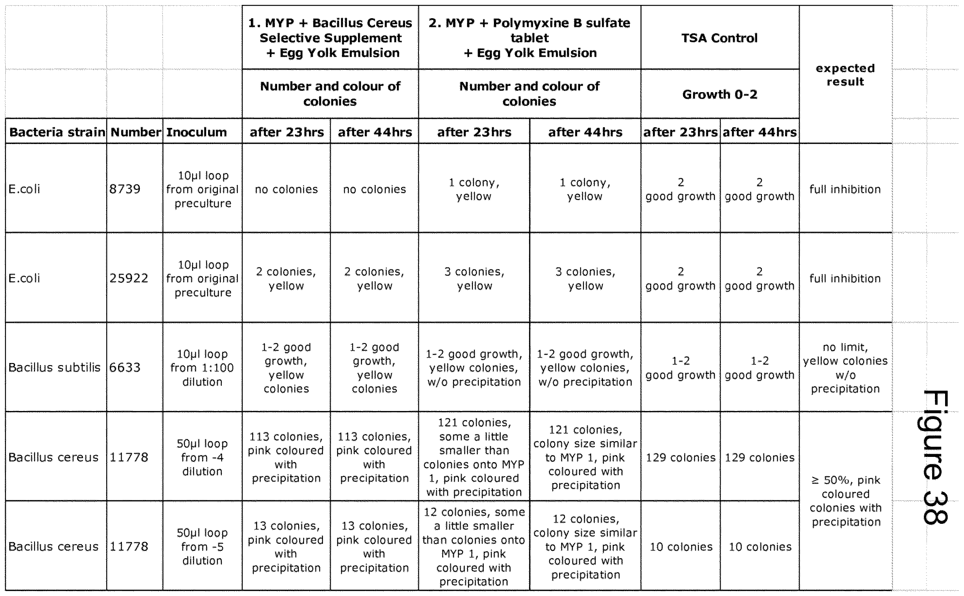

Figure 38 is a table showing the results of the cell culture assay according to Example 13.

The present invention is further directed to a method as defined in the claims for culturing cells whereby a 3D printed solid cell culture media form is used.

Thus the present invention can involve a method for culturing cells by
a) generating a solid cell culture media form by using 3D printing technologies
b) mixing said solid form with a liquid and the cells to be cultured, thereby dissolving the solid form
c) performing cell culture by incubating the mixture of step b)

In one embodiment, the solid form of a cell culture medium or solid cell culture media form is a base medium. In this case, the solid form is dissolved in a suitable solvent like water or an aqueous buffer. Further components like a gelling agent might be added. Then the cells to be cultured are added. Cells are cultured by incubating them in the cell culture medium under suitable conditions. The solid cell culture media form is suitable as base medium especially for applications in which different compositions of base media shall be tested. Due to 3D printing the composition of the solid cell culture media forms can be precisely defined and varied as needed. For example, the effect of the variation of the amount of a certain ingredient can be tested by printing several solid forms with a differing amount of said ingredient. In addition, one ingredient can be substituted by another. It is also possible and preferred to use a solid form as a base medium which has sustained release properties. The readily dissolving outer shell comprises all components that need to be present to start cell culture. The inner core comprises cell culture media ingredients that shall be released at a later stage of the cell culture.

It is also possible and preferred to use a solid cell culture media form as a base medium to start cell culture or as a media supplement which comprises at least two layers comprising a different composition of cell culture media ingredients. As described above, certain cell culture media ingredients interact with each other and typically need to be added to the cell culture separately, e.g. by including one in the base medium and one in a media supplement. By using a 3D printed solid form, the addition of one of the components in the form of a media supplement is not necessary any more. Both components can be present in different layers of the solid form so that they cannot interact with each other but can nevertheless be applied in one step.

In another embodiment the solid cell culture media form comprises at least two different layers. It might be used as base medium or as medium supplement. The at least two layers have a different composition. Some cell culture media ingredients have very different dissolution properties. While some ingredients are readily soluble in water, other ingredients are nearly insoluble in water but are easily soluble in other solvents like e.g. ethanol or DMSO. By preparing a solid cell culture media form with two or more layers all ingredients with similar solubility can be put in one layer. With this approach the amount of solvent can be significantly reduced compared to an approach in which all ingredients are dissolved in large amounts of a single type of solvent.

In another especially preferred embodiment the solid cell culture media form is preferably a media supplement. The media supplement can either be first mixed with a liquid base medium or a liquid and a dry powder or a solid form base medium or it can be added to the cell culture at any time during the cell culture.

In one embodiment, the media supplement comprises a cell culture media ingredient which is typically difficult to add in a defined amount. By providing the media supplement in a 3D printed solid form, the amount of the ingredient can be defined precisely. It is very easy for the user to just add the solid form to the base medium or the cell culture. Examples of such ingredients are trace elements.

In another embodiment, the media supplement comprises at least one ingredient that is not stable, is difficult to dissolve or has other specific properties that need to be attended to. If the ingredient is not stable or hygroscopic, the solid form might comprise a shell that does not contain said ingredient but that shields the core comprising said ingredient from the environment. Once the solid form is contacted with an aqueous solution, the shell will dissolve and set free the core.

In another embodiment, the media supplement comprises an ingredient whose dissolution properties need to be adjusted. Ingredients that are not sufficiently soluble if applied as powders or particles and typically need to be pre-dissolved separately can be added as solid form, whereby the solid form comprises a structure and/or composition that supports the dissolution of said ingredient. Examples of such ingredients are certain amino acids like cysteine and tyrosine as well as branched chain amino acids like valine, leucine and isoleucine.

In another preferred embodiment, the solid form media supplement comprises at least a core and a shell with a differing composition. It might also comprise more than one shell layer. This structure enables a controlled release of the ingredients. The cell culture media ingredients in the outer layer are released first. The release of the ingredients in the inner layers and/or the core is controlled by their composition and structure. By adding a media supplement in the form of a 3D printed solid form comprising a core shell structure once, the addition of several single doses of identical or different media supplements can be covered as all single doses are comprised in the one solid form. This makes the strategy of feeding or supplementing a cell culture much easier as ideally one solid cell culture media form can be added instead of several doses of conventional media supplements. This approach is valuable for fed batch processes as well as for microbiological applications. For example, for the detection of Listeria monocytogenes in food and environmental samples according to the FDA-BAM methodology as well as for the enumeration of Listeria monocytogenes in food samples by MPN, the addition of the selective agents is typically done after about four hours after starting the cell culture. For this, a media supplement is added. By either using a solid form base medium which comprises a sustained release core comprising the selective agent or by adding a solid form media supplement comprising a sustained release core comprising the selective agent when starting the cell culture, the additional step of adding the selective agent after 4 hours can be omitted. Details about the processes for the detection of Listeria monocytogenes in food and environmental samples as well as for the enumeration of Listeria monocytogenes in food samples can be found in the FDA Bacteriological Analytical Manual

Chapter 10 "Detection of Listeria monocytogenes in Foods and Environmental Samples, and Enumeration of Listeria monocytogenes in Foods", authors: Anthony D. Hitchins (ret.) and Karen Jinneman and Yi Chen, revision date: March 2017.

Performing a cell culture is known to a person skilled in the art. Typically, the medium is placed in a suitable container and inoculated with the cells or a sample potentially comprising said cells. Suitable containers are defined above. A sample can be any liquid, gaseous or solid entity that can be contacted with the cell culture medium. The sample can be a surface that is e.g. contacted with a contact plate comprising the cell culture medium. It can for example be a swap that is contacted with the cell culture medium or any liquid or solid that is put in or onto the medium.

The temperature of incubation of the medium to allow growth of cells is typically between 0 °C and 100 °C, more typically between 15 °C and 50 °C, still more typically between 20 and 45°C. The cell culture may for example be incubated at room temperature (around 20°), or at about 32.5°C.

Generally, after inoculation, the medium is incubated for a period of time to enable some growth of the cells.

For applications that aim the detection or enumeration of cells, this time can range from a minimum of hours to weeks. Generally, the incubation time is between about 1 and 14 days.

For applications in biopharmaceutical production, typically a fed-batch cell culture is performed. This might also last for several weeks. The aim in biopharmaceutical production typically is to develop high-titer cell culture processes to meet increasing market demands and reduce manufacturing costs. Beside the use of high-performing recombinant cell lines, improvements in cell culture media and process parameters are required to realize the maximum production potentials.

In a fed-batch process, a base medium supports initial growth and production, and the addition of media supplements, also called feed media one or more times during the cell culture, prevents depletion of nutrients and sustains the production phase. The media are chosen to accommodate the distinct metabolic requirements during different production phases. Process parameter settings - including feeding strategy and control parameters - define the chemical and physical environments suitable for cell growth and protein production.

Using the solid form media supplements generated in the process of the present invention for the first time offers an alternative cell culture media strategy for all types of cell culture. The media can be designed very specifically concerning their composition. They provide more flexibility when combining certain ingredients with different solubility or which can otherwise not be combined in different layers of the solid form. Including sustained release approaches offers new possibilities for feeding strategies. In addition the solid forms can be produced in any dosage and composition wanted. A user in need of a higher dosage can simply produce a larger solid form or add two or more solid forms which is much easier than opening two or more vials and adding two or more doses of liquid.

The present invention, without being limited thereby, is further illustrated by the following examples.

### Example 1 (Production of a layer containing laser energy absorbing material (1) or (1"))

195 g of Butylacetate
16 g of PVB (polyvinylbutyral, Pioloform,Wacker)
11 g Vestosint 2070
3g Aerosil 200
30 g of Sn(Sb)O~(d5o value<1,1µm) (Du Pont)

Polyvinylbutyral is dissolved in the initially introduced solvent Butylacetate and stirred well. The laser energy absorbing material Sn(Sb)O2 is subsequently stirred in, and a homogeneous paste is prepared. The amount of laser energy absorbing material is dependent on the energy absorption and should be set thereto. The paste is applied to a polyester film having a thickness of 5-250 µm, preferably 23µm, using a 30µm doctor blade (hand coater) and dried. The hot lamination can be carried out, for example, using a PE (polyethylene)-coated polypropylene film (Waloten film from Piitz) at about 140°C.

### Example 2 (Production of a layer containing laser energy absorbing material (1) or (1"))

200 g of Butylacetate
20 g of PVB (polyvinylbutyral, Piolo form, Wacker)
8 g Vestosint 2070
3 g Aerosil 200
25 g of gas black (d5O value&17 nm) (Special Black 6 from Degussa)

The processing is carried out as in Working Example 1. The laser energy absorbing material employed is gas black. The paste is applied to polyester films having a thickness of 5-250 µm using a 90 µm hand coater and dried.

A further polyester film or polypropylene film can be applied to the absorber layer by hot lamination (as described in Working Example 1).

### Example 3 (Production of a layer containing laser energy absorbing material (1) or (1"))

200 g of ε-Caprolactam
5 g of PVP (polyvinypyrrolidon)
10 g of Natrosol 250 GR
15 g Vestosint 2070
3g Aerosil 200
25 g of Carbon black powder

The processing is carried out as in Example 1. The paste is applied to polyester films having a thickness of 5-250 µm using a 90 µm hand coater or screenprinter with use of stainless-steel screen of 250mesh/inch, 25µm wire diameter, 25µm emulsion thickness. And finally dried in a convection oven at 50°C for 1 hour.

A further polyester film or polypropylene film can be applied to the absorber layer by hot lamination (as described in Working Example 1).

### Example 4 (Production of a layer containing laser energy absorbing material (1) or (1"))

200 g of Masterblend 50 (SICPA-AARBERG AG)
10 g of Iriodin Lazerflair 825 (particle size 20µm) (Merck KGaA)
100 g of ethyl acetate/ethanol (1 : 1)
5 g Vestosint 2070

The laser energy absorbing material Iriodin Lazerflair 825 is incorporated into the Masterblend 50 under gentle conditions and printed by gravure printing onto a polyester film having a thickness of 5-250 µm, preferably 23 µm. The desired viscosity can be set using the solvent mixture ethyl acetate/ethanol. The application rate is 0. 5-1 g/cm.

### Example 5 (Production of a layer containing laser energy absorbing material (1) or (1"))

The layer is produced from polyester already containing laser energy absorbing material by addition of 300 g of Sn(Sb)O2 having a particle size of <1 µm (Du Pont) to the polyester masterbatch (10 kg). Films having a layer thickness of 5-200 µm are subsequently produced. The finished film contains 0. 05-10% by weight of laser energy absorbing material, depending on the layer thickness.

### Example 6 (Production of a layer containing laser energy absorbing material (1) or (1"))

The layer is produced from polyester already containing laser energy absorbing material by addition of 330 g of Carbon Black having a particle size of <0,5 µm to the polyester masterbatch (10 kg). Films having a layer thickness of 5-200 µm are subsequently produced. The finished film contains 0. 05-10% by weight of laser energy absorbing material, depending on the layer thickness.

### Example 7 (Preparation of a tape with a cell culture media ingredient containing layer (2), in this case a mCCD Agar supplement)

2,1 g Soluplus ^{®}, 3g Cefoperazone Sodium Salt, 1g Amphotericin B and 43g PEG 6000 are added sequentially into a liquid mixture of 45 g water and 15 g ethanol, and stirred well with a high speed dissolver, resulting in a homogeneous paste. This paste is applied onto the PET film using a 100-micron hand coater, then dried in a convection oven at 50°C, 1013 mbar, for 10 minutes.

### Example 8 (Preparation of a tape with a cell culture media ingredient containing layer (2), in this case Bolton Broth Selective supplement)

2,1 g Soluplus ^{®}, 2g Cefoperazone Sodium Salt, 2g Vancomycin hydrochloride, 2g Trimethoprim Lactate Salt, 1g Amphotericin B and 43g PEG 6000 are added sequentially into a liquid mixture of 45 g water and 15 g ethanol, and stirred well with a high speed dissolver, resulting in a homogeneous paste. This paste is applied onto the PET film using a 100-micron hand coater, then dried in a convection oven at 50°C, 1013 mbar, for 10 minutes.

### Example 9 (Preparation of a tape with a cell culture media ingredient containing layer (2), in this case Preston Broth Selective supplement)

2,1 g Soluplus ^{®}, Polymyxin B Sulfate 2500 IU, 1g Rifampicin, 1g Trimethoprim Lactate Salt, 1g Amphotericin B and 43g PEG 6000 are added sequentially into a liquid mixture of 45 g water and 15 g ethanol, and stirred well with a high speed dissolver, resulting in a homogeneous paste. This paste is applied onto the PET film using a 100-micron hand coater, then dried in a convection oven at 50°C, 1013 mbar, for 10 minutes.

The broths to be supplemented with the supplements of examples 8 and 9 are often used in 225 ml volumes, and the supplements added (in form of a liquid suspension) immediately prior to use. Hence tablets sized for 225 ml (or multiples of 225 ml) are more convenient than vials. Rifampicin and Amphotericin B are not water soluble, which necessitates the use of organic solvents (e.g. Ethanol) for preparing the conventional liquid supplement. This solubility issue does not come up when using the 3D printing technology.

### Example 10 (Preparation of a tape with a cell culture media ingredient containing layer (2) comprising polymyxin)

3,2 g Soluplus ^{®}, 0,1g Polymyxin B Sulfate, 8,5g PEG 6000, 8,5g PEG 2000, 0,5g Magnesiumstearat and 0,5g SiO2 are added sequentially into a liquid mixture of 14 g water and 0,5 g ethanol, and stirred well with a high speed dissolver, resulting in a homogeneous paste. This paste is applied onto the PET film using a 100-micron hand coater, then dried in a convection oven at 50°C, 1013 mbar, for 10 minutes.

Preparation of one tablet with the tape needs 100 layers. Each layer has a layer-thickness of 30µm and a rectangular area of 1,2cm² (1,7cm × 0,7cm). Transfer of each layer onto a building-platform has been realized by a Nd-YAG Laser.

### Example 11 (preparation of liquid culture media)

To compare the performance of a conventional liquid supplement and a supplement used in the invention two supplements and the final media are prepared:

### 1. MYP + egg yolk emulsion + Bacillus cereus selective supplement (according to the state of the art)

Dissolve 21.5g MYP DCM in 450ml of demineralized water. Heat in boiling water, autoclave for 15 minutes at 121°C, cool down to 47-50°C in a waterbath, add 50ml of egg yolk emulsion (sterile), mix (magnetic stirrer), add one vial (5mg) of Bacillus cereus selective supplement (resolved in 1ml sterile demineralized Water). Mix. Prepare plates by hand.

### 2. MYP + egg yolk emulsion + Polymyxine B sulfate tablet (prepared according to Example 10)

Dissolve 5.57g MYP DCM in 116.64ml of demineralized Water. Heat in boiling water, autoclave for 15 minutes at 121°C, cool down to 47-50°C in a waterbath, add one tablet (1.296mg) of Polymyxine B Sulfate, flask is kept in the waterbath at 47-50°C and permanently mixed, after dissolution of tablet add 12.96ml of egg yolk emulsion. Mix. Prepare plates by hand.

Sources of ingredients/media:
MYP means DCM MYP Agar Base (mannitol egg yolk polymyxin) acc. ISO 7932; ISO 21871 and FDA-BAM (Merck, Germany, article number 1.05267.0500)
Egg yolk emulsion (Merck, Germany, article number 1.03784.0001)
Bacillus Cereus Selective Supplement (Merck, Germany, article number)1.09875.0010)
Polymyxine B sulfate for preparation of tablet (Xellia Pharmaceut., article number 2.77426.0000)
Appearance and pH values (target pH value 7,2 ±0.2) of the two media:

| | DCM base after autoclaving | MYP agar + Polymyxine | Final MYP agar (incl. egg emulsion) | Final pH |
|---|---|---|---|---|
| 1. | clear to weak opalescent | clear to weak opalescent | opaque, pale pink | 7,270 |
| 2. | clear to weak opalescent | strong turbid, several particles from tablet visible | opaque, pale pink | 7,235 |

### Example 12 (preparation of bacteria)

Precultures of bacteria strains are performed in 5ml TSB.

The following strains are used:

| | |
|---|---|
| - Bacillus cereus ATCC1 1778 | expected result: growth and recovery > 50% |
| - Bacillus subtilis ATCC6633 | expected result: growth and recovery. no limit |
| - E.coli ATCC 8739 | expected result: full inhibition |
| - E.coli ATCC25922 | expected result: full inhibition |

### Example 13 (cell culture assay performance and results)

Inoculation of cells precultures according to Example 12 is performed as follows:
Bacillus cereus ATCC11778: 50µl from -4 and -5 dilution onto agar plates with Spiralplater
Bacillus subtilis ATCC6633: make a 1:100 dilution from original preculture and spread with a 10µl loop onto agar plates
E.coli ATCC 8739: spread from the original preculture with a 10µl loop onto agar plates
E.coli ATCC25922: spread from the original preculture with a 10µl loop onto agar plates

### Dilution Media: sodium chloride peptone broth (buffered)

The plates are incubated for 44 hours at 31°C aerobically. The detailed results are shown in Figure 38. One can see that the supplement that has been prepared with the media tablet performs equally compared to the liquid supplement according to the state of the art.

### Example 14 (Preparation of medium for the separation layer (13))

125 g of 1,2-Propandiole
1 g PEG 400
15 g Carnauba wax
5 g of Witepsol E85
34 g of Lactose
1 g of Povidone K90
13 g of Carboxymethylcellulose
0,7 g of Magnesiumstearat
0,5 g of Siliciumdioxide (Aerosil 200 Pharma)

The substances are added successive into the liquid mixture of 1,2-Propandiol and stirred well with high speed dissolver, and a homogeneous paste is prepared. The pasty mixture is homogenized by a tree roll mill. The paste is applied to polyester films having a thickness of 5-250 pm using a 90 pm hand coater and dried in a convection oven at 10mbar (inside pressure of the drying chamber) for 1 hour.

### Example 15 (Preparation of medium for the separation layer (13))

125 g of 1,2-Propandiole
1 g PEG 400
15 g Carnauba wax
34 g of Lactose
1 g of Povidone K90
13 g of Carboxymethylcellulose
0,7 g of Magnesiumstearat
0,5 g of Siliciumdioxide (Aerosil 200 Pharma)

The substances are added successive into the liquid mixture of 1,2-Propandiol and stirred well with high speed dissolver, and a homogeneous paste is prepared. The pasty mixture is finally homogenized by a tree roll mill. The paste is applied to polyester films having a thickness of 5-250 pm using a 90 pm hand coater and dried in a convection oven at 10mbar (inside pressure of the drying chamber) for 1 hour.

### Example 16 (Preparation of medium for the separation layer (13))

125g of 1,2-Propandiole
1 g PEG 400
20 g Carnauba wax
5 g of Witepsol H37
34 g of Lactose
1 g of Povidone K90
13 g of Carboxymethylcellulose
0,7g of Magnesiumstearat
0,5 g of Siliciumdioxide (Aerosil 200 Pharma)

The substances are added successive into the liquid mixture of 1,2-Propandiol and stirred well with high speed dissolver, and a homogeneous paste is prepared. The pasty mixture is homogenized by a tree roll mill. The paste is applied to polyester films having a thickness of 5-250 pm using a 90 pm hand coater and dried in a convection oven at 10mbar (inside pressure of the drying chamber) for 1 hour.

### Example 17 (Preparation of medium for the separation layer (13))

125 g of 1,2-Propandiole
1 g PEG 400
25 g Carnauba wax
10 g of Witepsol W31
34 g of Lactose
1 g of Povidone K90
13 g of Carboxymethylcellulose
0,7 g of Magnesiumstearat
0,5 g of Siliciumdioxide (Aerosil 200 Pharma)

The substances are added successive into the liquid mixture of 1,2-Propandiol and stirred well with high speed dissolver, and a homogeneous paste is prepared. The pasty mixture is homogenized by a tree roll mill. The paste is applied to polyester films having a thickness of 5-250 pm using a 90 pm hand coater and dried in a convection oven at 10mbar (inside pressure of the drying chamber) for 1 hour.

### Example 18 (Preparation of medium for the separation layer (13))

150 g of 1,2-Propandiole
1 g PEG 400
15 g Carnauba wax
5 g of Witepsol E85
34 g of Lactose
5 g of Povidone K90
13 g of Carboxymethylcellulose
0,7 g of Magnesiumstearat
0,5 g of Siliciumdioxide (Aerosil 200 Pharma)

The substances are added successive into the liquid mixture of 1,2-Propandiol and stirred well with high speed dissolver, and a homogeneous paste is prepared. The pasty mixture is homogenized by a tree roll mill. The paste is applied to polyester films having a thickness of 5-250 pm using a 90 pm hand coater and dried in a convection oven at 10mbar (inside pressure of the drying chamber) for 1 hour.

### Example 19 (Preparation of medium for the adhesion layer (14))

150g of 1,2-Propandiole
1 g PEG 400
15 g Carnauba wax
34 g of Lactose
6 g of Povidone K90
13 g of Carboxymethylcellulose
0,7 g of Magnesiumstearat
0,5 g of Siliciumdioxide (Aerosil 200 Pharma)

The substances are added successive into the liquid mixture of 1,2-Propandiol and stirred well with high speed dissolver, and a homogeneous paste is prepared. The pasty mixture is homogenized by a tree roll mill. The paste is applied to polyester films having a thickness of 5-250 pm using a 90 pm hand coater and dried in a convection oven at 10mbar (inside pressure of the drying chamber) for 1 hour.

### Example 20 (Preparation of medium for the adhesion layer (14))

150 g of 1,2-Propandiole
1 g PEG 400
20 g Carnauba wax
5 g of Witepsol H37
34 g of Lactose
7 g of Povidone K90
13 g of Carboxymethylcellulose
0,7 g of Magnesiumstearat
0,5 g of Siliciumdioxide (Aerosil 200 Pharma)

The substances are added successive into the liquid mixture of 1,2-Propandiol and stirred well with high speed dissolver, and a homogeneous paste is prepared. The pasty mixture is homogenized by a tree roll mill. The paste is applied to polyester films having a thickness of 5-250 pm using a 90 pm hand coater and dried in a convection oven at 10mbar (inside pressure of the drying chamber) for 1 hour.

### Example 21 (Preparation of medium for the adhesion layer (14))

150 g of 1,2-Propandiole
1 g PEG 400
25 g Carnauba wax
10 g of Witepsol W31
34 g of Lactose
7 g of Povidone K90
13 g of Carboxymethylcellulose
0,7 g of Magnesiumstearat
0,5 g of Siliciumdioxide (Aerosil 200 Pharma)

The substances are added successive into the liquid mixture of 1,2-Propandiol and stirred well with high speed dissolver, and a homogeneous paste is prepared. The pasty mixture is homogenized by a tree roll mill. The paste is applied to polyester films having a thickness of 5-250 pm using a 90 pm hand coater and dried in a convection oven at 10mbar (inside pressure of the drying chamber) for 1 hour.

### Example 22 (Production of a composite layer (3) (Figure 4))

The support film (1') and laser energy absorbing layer (1") (Examples 1-6) is placed together with the support film (1‴) and laminated together with the aid of a hot laminator. The heatable roll is set here to a temperature of 140-175°C. After the hot lamination, the two films are strongly bonded to one another. If a PE-coated polypropylene film (Waloten film from Putz) is used, the lamination can be carried out at about 140°C. Finally the cell culture media ingredient containing layer (2) is applied (Examples 7-13) to the underneath side of the laminated support film (1‴).

### Example 23 (Production of a composite layer (3) (Figure 6))

The support film (1') and laser energy absorbing layer (1 ") (Examples 1-6) is placed together with the support film (1‴) and laminated together with the aid of a hot laminator. The heatable roll is set here to a temperature of 140-175°C. After the hot lamination, the two films are strongly bonded to one another. If a PE-coated polypropylene film (Waloten film from Putz) is used, the lamination can be carried out at about 140°C. The separation layer (13) is applied (Examples 14-17) to the underneath side of the laminated support film (1‴). The cell culture media ingredient containing layer (2) (Examples 7-13) is applied to the separation layer. Finally the adhesion layer (14) is applied (Examples 18-21) to the cell culture media ingredient containing layer (2).

### Example 24 (Production of a composite layer (3) (Figure 5))

The medium for the cell culture media ingredient containing layer (2) (Examples 7-13) is applied to the laser energy absorbing layer (1) in a layer thickness of 225 µm (Examples 5-6) and dried.

### Example 25 (Production of a composite layer (3) (Figure 14))

The medium for the cell culture media ingredient containing layer (2) (Examples 7-13) is applied to the underneath side of a PET film (thickness: 5,12, 15, 19, 23, 36, 50 and 200 µm) in a layer thickness of 10 µm up to 500 µm, and a laser energy absorbing layer (Examples 1-6) is printed onto the laser side (upper side) in a layer thickness of 0,7-15 µm.

### Example 26 (Preparation of medium for the separation layer (13))

15g of Water
5 g of Ethanol
10g of Povidone
6g of Polyethylenglycol 6000
6g of Kolliphor P188
0,2 g of Magnesiumstearat
0,1 g of Siliciumdioxide (Aerosil 200 Pharma)

The substances are added successive into the liquid mixture of Water/Ethanol and stirred well with high speed dissolver, and a homogeneous paste is prepared. The paste is applied to polyester films having a thickness of 5-100 micron using a 30 micron hand coater and dried in a convection oven at 50°C, 10mbar (inside pressure of the drying chamber) for 5 minutes.

### Example 28 (Detailed description of the operation of a preferred embodiment of an apparatus usable for the process to be used for the present invention (Figure 36))

### Step 1

A cassette system is implemented into the 3D printer device which includes a tape (3') of the composite layer (3), which is shown in FIG. 7.

### Step 2

A protection tape (10) is placed onto the mounting plate (6).

### Step 3

The mounting plate (6) is adjusted on zero level for the start position.

### Step 4

The composite layer (3) is forwarded by the step engine to the start position.

### Step 5

Positioner roll (4') and (4") drops down to press the composite layer (3) onto the mounting plate (6).

### Step 6

The tension of the composite layer between the two rolls (11) and (11 ') is adjusted to an appropriate tension and the vacuum pump (9) is switched on.

### Step 7

The laser scans a defined area thereby transferring the cell culture media ingredient containing layer (2) onto the mounting plate (6).

### Step 8

The positioner rolls (4') and (4") are moved up for complete liftoff of the cell culture media ingredient layer (2).

### Step 9

The composite layer (3) (provided as tape (3')) is scrolled forward by the stepper motor thereby positioning new (intact) composite layer (3) to the scanning area.

### Step 10

The mounting plate (6) is moved down with a height that is identical to the thickness of the cell culture media ingredient layer (2).

### Step 11

Positioner roll (4') and (4") drops down to press the composite layer (3) onto the mounting plate (6).

### Step 12

Repeating the process steps 6 to 11 as often as needed to build up the solid cell culture media form;

### Step 13

Switch off the vacuum pump (9) and removal of the solid cell culture media form from the mounting plate (6).

### Example 29 (Production of a solid form in accordance with example 28)

Starting with production of a composite layer (3) (Figure 7))

### Step 1

### Preparation of the medium for separation layer (13)

15g of Water
5 g of Ethanol
10g of Povidone
6g of Polyethylenglycol 6000
6g of Kolliphor P188
0.1 g of Siliciumdioxide (Aerosil 200 Pharma)

The substances are added successive into the liquid mixture of Water/Ethanol and stirred well with high speed dissolver, and a homogeneous paste is prepared. The paste is applied to polyester films having a thickness of 50 micron using a 30 micron hand coater and dried in a convection oven at 50°C, 1013mbar for 5minutes.

### Step 2

### Preparation of a medium for a cell culture media ingredient containing layer (2))

45g of Water
15 g of Ethanol
9g of Povidone
12g of Sorbitol
24g of Trimethoprim Lactate
9 g of Magnesiumstearat
0,1 g of Siliciumdioxide (Aerosil 200 Pharma)

The substances are added successive into the liquid mixture of Water/Ethanol and stirred well with high speed dissolver, and a homogeneous paste is prepared. The paste for the cell culture media ingredient containing layer (2) is applied to the separation layer having a thickness of 70 micron using a 200 micron hand coater and dried in a convection oven at 50°C, 1013 mbar for 10 minutes.

### Step 3

Cutting of the composite layer (3) in stripes of 2 cm width thereby obtaining tapes (3') that are rolled on a core roll (11).

### Step 4

Assembling the core roll (11) in a cassette system (16) and connecting it with the recoil roll (11').

### Step 5

Implementation of the cassette system (16) to the configuration shown in FIG. 36

### Further Steps

Conducting Steps 2 to 13 set forth in Example 28. As a result a solid form having total weight of 400mg containing 39% (w/w) Ibuprofen is obtained.

### Experimental conditions

Composite layer (1) with a separation layer (13), cell culture media ingredient containing layer (2) and adhesion layer (14) (FIGS. 4-17 and 24-29) are employed for the method of the present invention with the aid of the following laser types and parameters:
a) Nd:YAG (cw mode)

| 12 watt laser | Trumpf laser |
|---|---|
| Nd: | YAG (1064 and 532 nm) |
| Laser intensity: | 10 - 100%, cw mode |
| Speed: | 100 - 5000 mm/s |
| Line width: | 0,1mm |
| Line gap: | 0,05mm |

b) Nd:YVOa laser (cw mode, pulsed)

| 16 watt laser | Rofin Sinar |
|---|---|
| Nd: | YVO4 (1064 nm) |
| Laser intensity: | 20 - 90%, cw mode, pulsed |
| Pulse frequency: | 10 - 100 kHz |
| Speed: | 400-2000 mm/s |

c) Nd: YAG laser (pulsed)

| 60 watt laser | Baasel |
|---|---|
| Nd: | YAG (1064 nm) |

d) Nd:YAG (pulse mode)

| 12 watt laser | Trumpf laser |
|---|---|
| Nd: | YAG (1064nm) |
| Laser intensity: | 10 - 100%, pulse mode |
| Speed: | 100 - 5000 mm/s |
| Pulse frequence: | 5kHz |
| Line width: | 0,1mm |
| Line gap: | 0,05mm |

### Lamp current 16 A, pulsed mode

| | |
|---|---|
| Pulse frequency: | 20.000 Hz |
| Speed: | 200 mm/s |
| Wobbler frequency: | 16 Hz |
| Pulse duration: | 0.05 ms |

Compared with the printing in cw mode of small structures (linewidth <1mm or dot diameter <1mm), the final 3D printing result in pulsed mode are distinguished by greater edge sharpness and smoother surface at the laser printing points.

## Claims

1. A process for culturing cells by
a) generating a solid form of a cell culture medium comprising at least one cell culture media ingredient by using 3D printing technologies
b) mixing said solid form with a liquid and the cells to be cultured, thereby dissolving the solid form
c) performing cell culture by incubating the mixture of step b)

2. A process according to claim 1, whereby the solid form is a tablet.

3. A process according to claim 1 or claim 2, whereby the solid form comprises at least two layers with differing composition.

4. A process according to claim 1 or claim 2, whereby the solid form comprises a core and a shell with differing composition.

5. A process according to one or more of claims 1 to 4, whereby, when mixing the solid form with a liquid a part of the solid form is dissolved slower compared to another part of the solid form.

6. A process according to one or more of claims 1 to 5, whereby the solid form comprises 4-methylumbilliferyl-phosphate disodium, Acriflavine, Amphotericin B, Ammonium-Iron(III)-citrate, Brilliant green, Calcium carbonate, Cefiximide, Cefoperazone, Cefotetan, Cefsulodin, Ceftazimide, Cephalotin, Cetrimide, Colistin sulfate, Cyclohexidine, D-cycloserine, Fosfomycin, Fucidin, Irgasan, L-α-Phosphatidylinositol, Lithium mupirocin, Nalidixic acid, Novobiocin, Ox bile, Oxytetracycline, Polymyxin B sulfate, Potassium tellurite, Potassium tetrathionate, Rifampicin, Trimethoprim lactate salt or Vancomycin or combinations thereof.

7. A process according to one or more of claims 1 to 6, whereby the solid form comprises one or more amino acids.

8. A process according to one or more of claims 1 to 7, whereby step b) is performed by first mixing said solid form with a liquid and then adding the cells.

9. A process according to one or more of claims 1 to 7, whereby step b) is performed by mixing said solid form with a liquid that already comprises the cells.

10. A process according to one or more of claims 1 to 7, whereby the cell culture performed in step c) is a fed batch cell culture.

11. A process according to one or more of claims 1 to 7, whereby the cell culture performed in step c) is a cell culture for detection and/or enumeration of certain cells.

12. A process according to one or more of claims 1 to 7, whereby the 3D printing technology used in step a) is a contactless 3D laser printing process.

13. A process according to claim 12, whereby the printing process used in step a) comprises the steps
(a) positioning a composite layer (3) comprising a laser energy absorbing layer (1) and a layer that contains at least one cell culture media ingredient (2) between a plate (4) that is permeable for a laser beam that can be activated by a source of laser energy (5) and a mounting plate (6) whereat the layer of the composite containing at least one cell culture media ingredient (2) is positioned opposite to the source of laser energy (5) and is facing to the mounting plate (6);
(b) lowering the mounting plate (6) to shape an interspace (8) between the composite layer (3) comprising a cell culture media ingredient containing layer (2) and the mounting plate (6);
(c) transferring by action of laser beam from the source of laser energy (5) the cell culture media ingredient containing layer (2) of the composite (3) onto the mounting plate (6);
(d) repeating steps (a), (b) and (c) as often as needed to build up the solid form of a cell culture medium;
(e) removing the solid form of a cell culture medium from the mounting plate.

14. A process according to claim 12, whereby the printing process used in step a) comprises the steps
(a) positioning a composite layer (3) comprising a laser energy absorbing layer (1) and a layer that contains at least one cell culture media ingredient (2) between between a plate (4) that is permeable for a laser beam that can be activated by a source of laser energy (5) and a mounting plate (6) comprising at least one area (7) that is movable in vertical direction (z axis) relative to the mounting plate whereat the layer of the composite containing at least one cell culture media ingredient (2) is positioned opposite to the source of laser energy (5) and is facing to the mounting plate (6);
(b) lowering the movable area (7) relative to the mounting plate (6) to shape an interspace (8') between the composite layer (3) comprising a cell culture media ingredient containing layer (2) and the movable area (7) of the mounting plate (6);
(c) transferring by action of laser beam from the source of laser energy (5) the cell culture media ingredient containing layer (2) of the composite (3) into the interspace (8') shaped by the movable area (7) that was lowered vertically relative to the mounting plate (6);
(d) repeating steps (a), (b) and (c) as often as needed to build up the solid form of a cell culture medium;
(e) removing the solid form of a cell culture medium from the mounting plate.

## Patentansprüche

1. Verfahren zum Kultivieren von Zellen durch
a) Erzeugen einer festen Form eines Zellkulturmediums, das mindestens einen Zellkulturmedienbestandteil umfasst, unter Verwendung von 3D-Drucktechnologien,
b) Mischen der festen Form mit einer Flüssigkeit und den zu kultivierenden Zellen, wodurch sich die feste Form löst,
c) Durchführen einer Zellkultur durch Inkubieren des Gemischs aus Schritt b).

2. Verfahren nach Anspruch 1, wobei die feste Form eine Tablette ist.

3. Verfahren nach Anspruch 1 oder Anspruch 2, wobei die feste Form mindestens zwei Schichten mit unterschiedlicher Zusammensetzung umfasst.

4. Verfahren nach Anspruch 1 oder Anspruch 2, wobei die feste Form einen Kern und eine Hülle mit unterschiedlicher Zusammensetzung umfasst.

5. Verfahren nach einem oder mehreren der Ansprüche 1 bis 4, wobei beim Mischen der festen Form mit einer Flüssigkeit ein Teil der festen Form im Vergleich zu einem anderen Teil der festen Form langsamer gelöst wird.

6. Verfahren nach einem oder mehreren der Ansprüche 1 bis 5, wobei die feste Form 4-Methylumbilliferylphosphat-Dinatrium, Acriflavin, Amphotericin B, Ammonium-Eisen(III)-citrat, Brillantgrün, Calciumcarbonat, Cefixim, Cefoperazon, Cefotetan, Cefsulodin, Ceftazidim, Cefalotin, Cetrimid, Colistinsulfat, Cyclohexidin, D-Cycloserin, Fosfomycin, Fucidin, Irgasan, L-α-Phosphatidylinositol, Lithiummupirocin, Nalidixinsäure, Novobiocin, Ochsengalle, Oxytetracyclin, Polymyxin-B-sulfat, Kaliumtellurit, Kaliumtetrathionat, Rifampicin, Trimethoprim-Lactatsalz oder Vancomycin oder Kombinationen davon umfasst.

7. Verfahren nach einem oder mehreren der Ansprüche 1 bis 6, wobei die feste Form eine oder mehrere Aminosäuren umfasst.

8. Verfahren nach einem oder mehreren der Ansprüche 1 bis 7, wobei Schritt b) durchgeführt wird, indem zunächst die feste Form mit einer Flüssigkeit gemischt wird und dann die Zellen zugegeben werden.

9. Verfahren nach einem oder mehreren der Ansprüche 1 bis 7, wobei Schritt b) durchgeführt wird, indem die feste Form mit einer Flüssigkeit, die bereits die Zellen enthält, gemischt wird.

10. Verfahren nach einem oder mehreren der Ansprüche 1 bis 7, wobei die in Schritt c) durchgeführte Zellkultur eine Fed-Batch-Zellkultur ist.

11. Verfahren nach einem oder mehreren der Ansprüche 1 bis 7, wobei die in Schritt c) durchgeführte Zellkultur eine Zellkultur zum Nachweis und/oder zur Zählung bestimmter Zellen ist.

12. Verfahren nach einem oder mehreren der Ansprüche 1 bis 7, wobei die in Schritt a) verwendete 3D-Drucktechnologie ein kontaktloses 3D-Laserdruckverfahren ist.

13. Verfahren nach Anspruch 12, wobei das in Schritt a) verwendete Druckverfahren die Schritte
(a) Positionieren einer Verbundschicht (3), umfassend eine Laserenergie absorbierende Schicht (1) und eine Schicht, die mindestens einen Zellkulturmedienbestandteil (2) enthält, zwischen einer Platte (4), die für einen Laserstrahl, der durch eine Laserenergiequelle (5) aktiviert werden kann, durchlässig ist, und einer Montageplatte (6), wobei die Schicht des Verbunds, die mindestens einen Zellkulturmedienbestandteil (2) enthält, gegenüber der Laserenergiequelle (5) positioniert ist und der Montageplatte (6) zugewandt ist;
(b) Absenken der Montageplatte (6), um einen Zwischenraum (8) zwischen der Verbundschicht (3), die eine einen Zellkulturmedienbestandteil enthaltende Schicht (2) umfasst, und der Montageplatte (6) zu bilden;
(c) Übertragen der den Zellkulturmedienbestandteil enthaltenden Schicht (2) des Verbunds (3) auf die Montageplatte (6) durch Einwirkung eines Laserstrahls von der Laserenergiequelle (5);
(d) Wiederholen der Schritte (a), (b) und (c) so oft wie nötig, um die feste Form eines Zellkulturmediums aufzubauen;
(e) Entfernen der festen Form eines Zellkulturmediums von der Montageplatte
umfasst.

14. Verfahren nach Anspruch 12, wobei das in Schritt a) verwendete Druckverfahren die Schritte
(a) Positionieren einer Verbundschicht (3), umfassend eine Laserenergie absorbierende Schicht (1) und eine Schicht, die mindestens einen Zellkulturmedienbestandteil (2) enthält, zwischen einer Platte (4), die für einen Laserstrahl, der durch eine Laserenergiequelle (5) aktiviert werden kann, durchlässig ist, und einer Montageplatte (6) mit mindestens einem Bereich (7), der in vertikaler Richtung (z-Achse) relativ zur Montageplatte beweglich ist, wobei die Schicht des Verbunds, die mindestens einen Zellkulturmedienbestandteil (2) enthält, gegenüber der Laserenergiequelle (5) positioniert ist und der Montageplatte (6) zugewandt ist;
(b) Absenken des beweglichen Bereichs (7) relativ zur Montageplatte (6), um einen Zwischenraum (8') zwischen der Verbundschicht (3), die eine einen Zellkulturmedienbestandteil enthaltende Schicht (2) umfasst, und dem beweglichen Bereich (7) der Montageplatte (6) zu bilden;
(c) Übertragen der den Zellkulturmedienbestandteil enthaltenden Schicht (2) des Verbunds (3) in den Zwischenraum (8'), der durch den beweglichen Bereich (7) gebildet wird, der relativ zur Montageplatte (6) vertikal abgesenkt wurde, durch Einwirkung eines Laserstrahls von der Laserenergiequelle (5);
(d) Wiederholen der Schritte (a), (b) und (c) so oft wie nötig, um die feste Form eines Zellkulturmediums aufzubauen;
(e) Entfernen der festen Form eines Zellkulturmediums von der Montageplatte
umfasst.

## Revendications

1. Procédé de culture de cellules, par
a) la génération d'une forme solide d'un milieu de culture cellulaire comprenant au moins un ingrédient de milieu de culture cellulaire, en utilisant des technologies d'impression 3D,
b) le mélange de ladite forme solide avec un liquide et les cellules à cultiver, solubilisant ainsi la forme solide,
c) la mise en oeuvre de la culture cellulaire par l'incubation du mélange de l'étape b).

2. Procédé selon la revendication 1, dans lequel la forme solide est un comprimé.

3. Procédé selon la revendication 1 ou la revendication 2, dans lequel la forme solide comprend au moins deux couches de composition différente.

4. Procédé selon la revendication 1 ou la revendication 2, dans lequel la forme solide comprend un coeur et une enveloppe de composition différente.

5. Procédé selon l'une ou plusieurs parmi les revendications 1 à 4, dans lequel, lors du mélange de la forme solide avec un liquide, une partie de la forme solide est solubilisée plus lentement qu'une autre partie de la forme solide.

6. Procédé selon l'une ou plusieurs parmi les revendications 1 à 5, dans lequel la forme solide comprend du phosphate de 4-méthyl-umbilliféryle disodique, de l'acriflavine, de l'amphotéricine B, du citrate d'ammonium-fer(III), du vert brillant, du carbonate de calcium, du céfixime, de la céfopérazone, du céfotétan, de la cefsulodine, du ceftazidime, de la céfalotine, du cétrimide, du sulfate de colistine, de la cyclohexidine, de la D-cyclosérine, de la fosfomycine, de la fucidine, de l'irgasan, du L-α-phosphatidyl-inositol, de la mupirocine de lithium, de l'acide nalidixique, de la novobiocine, de la bile de boeuf, de l'oxytétracycline, du sulfate de polymyxine B, du tellurite de potassium, du tétrathionate de potassium, de la rifampicine, un sel de lactate de triméthoprime ou de la vancomycine, ou des combinaisons de ceux-ci.

7. Procédé selon l'une ou plusieurs parmi les revendications 1 à 6, dans lequel la forme solide comprend un ou plusieurs acides aminés.

8. Procédé selon l'une ou plusieurs parmi les revendications 1 à 7, dans lequel l'étape b) est mise en oeuvre en mélangeant d'abord ladite forme solide avec un liquide puis en ajoutant les cellules.

9. Procédé selon l'une ou plusieurs parmi les revendications 1 à 7, dans lequel l'étape b) est mise en oeuvre en mélangeant ladite forme solide avec un liquide qui comprend déjà les cellules.

10. Procédé selon l'une ou plusieurs parmi les revendications 1 à 7, dans lequel la culture cellulaire mise en oeuvre dans l'étape c) est une culture cellulaire à alimentation discontinue.

11. Procédé selon l'une ou plusieurs parmi les revendications 1 à 7, dans lequel la culture cellulaire mise en oeuvre dans l'étape c) est une culture cellulaire pour la détection et/ou le dénombrement de certaines cellules.

12. Procédé selon l'une ou plusieurs parmi les revendications 1 à 7, dans lequel la technologie d'impression 3D utilisée à l'étape a) est un procédé d'impression laser 3D sans contact.

13. Procédé selon la revendication 12, dans lequel le procédé d'impression utilisé à l'étape a) comprend les étapes
(a) de positionnement d'une couche composite (3) comprenant une couche absorbant l'énergie laser (1) et une couche qui contient au moins un ingrédient de milieu de culture cellulaire (2) entre une plaque (4) perméable à un faisceau laser pouvant être activé par une source d'énergie laser (5) et une plaque de montage (6), la couche du composite contenant au moins un ingrédient de milieu de culture cellulaire (2) étant positionnée à l'opposé de la source d'énergie laser (5) et faisant face à la plaque de montage (6) ;
(b) d'abaissement de la plaque de montage (6) afin de façonner un espace intermédiaire (8) entre la couche composite (3) comprenant une couche contenant un ingrédient de milieu de culture cellulaire (2) et la plaque de montage (6) ;
(c) de transfert, sous l'action du faisceau laser provenant de la source d'énergie laser (5), de la couche contenant un ingrédient de milieu de culture cellulaire (2) du composite (3) sur la plaque de montage (6) ;
(d) de répétition des étapes (a), (b) et (c) aussi souvent que nécessaire afin de constituer la forme solide d'un milieu de culture cellulaire ;
(e) de retrait de la forme solide d'un milieu de culture cellulaire de la plaque de montage.

14. Procédé selon la revendication 12, dans lequel le procédé d'impression utilisé à l'étape a) comprend les étapes
(a) de positionnement d'une couche composite (3) comprenant une couche absorbant l'énergie laser (1) et une couche qui contient au moins un ingrédient de milieu de culture cellulaire (2) entre une plaque (4) perméable à un faisceau laser pouvant être activé par une source d'énergie laser (5) et une plaque de montage (6) comprenant au moins une zone (7) mobile dans la direction verticale (axe z) par rapport à la plaque de montage, la couche du composite contenant au moins un ingrédient de milieu de culture cellulaire (2) étant positionnée à l'opposé de la source d'énergie laser (5) et faisant face à la plaque de montage (6) ;
(b) d'abaissement de la zone mobile (7) par rapport à la plaque de montage (6) afin de façonner un espace intermédiaire (8') entre la couche composite (3) comprenant une couche contenant un ingrédient de milieu de culture cellulaire (2) et la zone mobile (7) de la plaque de montage (6) ;
(c) de transfert, sous l'action du faisceau laser provenant de la source d'énergie laser (5), de la couche contenant un ingrédient de milieu de culture cellulaire (2) du composite (3) dans l'espace intermédiaire (8') façonné par la zone mobile (7) qui a été abaissée verticalement par rapport à la plaque de montage (6) ;
(d) de répétition des étapes (a), (b) et (c) aussi souvent que nécessaire afin de constituer la forme solide d'un milieu de culture cellulaire ;
(e) de retrait de la forme solide d'un milieu de culture cellulaire de la plaque de montage.
